# EUROPEAN PATENT SPECIFICATION

(11) **EP 1 854 881 B1**
(45) Date of publication and mention of the grant of the patent: **02.06.2010**
(21) Application number: 06712765.4
(22) Date of filing: 01.02.2006
(51) Int. Cl.: C12N 15/09, C07K 14/47, C07K 16/18, C12N 1/15, C12N 1/19, C12N 1/21, C12N 5/10, C12P 21/02, C12Q 1/02, C12Q 1/68, G01N 33/48, G01N 33/53

(54) **METHODS FOR IDENTIFYING PURKINJE CELLS USING THE Corl2 GENE AS A TARGET**
VERFAHREN ZUR IDENTIFIZIERUNG VON PURKINJE-ZELLEN UNTER VERWENDUNG DES CORL2-GENS ALS ZIEL
PROCEDES D'IDENTIFICATION D'UN GENE Corl2 CIBLANT LES CELLULES DE PURKINJE

(30) Priority: 02.02.2005 JP 2005026970; 09.02.2005 JP 2005033704
(43) Date of publication of application: 14.11.2007
(73) Proprietor: Eisai R&D Management Co., Ltd., Tokyo 112-8088 (JP)
(72) Inventor: ONO, Yuichi, c/o KAN Research Institute, Inc., Kyoto-shi, Kyoto 600-8815 (JP); NAKATANI, Tomoya, Kyoto-shi, Kyoto 600-8815 (JP)
(74) Representative: Vossius & Partner
(86) International application number: PCT/JP2006/301622
(87) International publication number: WO 2006/082826

(56) References cited:
- DATABASE EMBL [Online] 13 February 2003 (2003-02-13), "Mus musculus BAC clone RP23-440H11 from chromosome 18, complete sequence." XP002488248 retrieved from EBI accession no. EMBL:AC139757 Database accession no. AC139757
- DATABASE EMBL [Online] 16 March 2002 (2002-03-16), "UI-M-CG0p-bqz-e-07-0-UI.r1 NIH_BMAP_Ret4_S2 Mus musculus cDNA clone UI-M-CG0p-bqz-e-07-0-UI 5', mRNA sequence." XP002488249 retrieved from EBI accession no. EMBL:BM942054 Database accession no. BM942054
- MIZUHARA E. ET AL.: 'Corl1, a novel neuronal lineage-specific transcriptional corepressor for the homeodomain transcription factor Lbx1' J. BIOL. CHEM. vol. 280, no. 5, 04 November 2004, pages 3644 - 3655, XP003002260
- DATABASE GENBANK [Online] CARNINCI P. AND HAYASHIZAKI Y.: 'High-efficiency full-lenght cDNA cloning', XP003002261 Retrieved from NCBI Database accession no. (AK049035)
- RONG Y. ET AL.: 'Identification of candidate Purkinje cell-specific markers by gene expression profiling in wild-type and pcd(3J) mice' MOL. BRAIN RES. vol. 132, no. 2, 2004, pages 128 - 145, XP004669597
- OHAMA E. ET AL.: 'Hito Shono Purkinje Saibo no Hassei to Hensei' SHINKEI SHINPO vol. 31, no. 2, 1987, pages 255 - 269, XP003002262

### SEQUENCE LISTING

<110> EISAI C0., LTD.
<120> METHOD OF IDENTIFYING PURKINJE CELL TARGETING Corl2 GENE
<130> E1-A0409P
<150> JP 2005-26970
   <151> 2005-02-02
<150> JP 2005-33704
   <151> 2005-02-09
<160> 32
<170> PatentIn version 3.3
<210> 1
   <211> 1008
   <212> PRT
   <213> Mus musculus
<400> 1
   Met Ala Ser Ser Pro Leu Pro Gly Pro Asn Asp Ile Leu Leu Ala Ser
   1 5 10 15
   Pro Ser Ser Ala Phe Gln Pro Asp Ala Leu Ser Gln Pro Arg Pro Gly
   20 25 30
   His Ala Asn Leu Lys Pro Asn Gln Val Gly Gln Val Ile Leu Tyr Gly
   35 40 45
   Ile Pro Ile Val Ser Leu Val Ile Asp Gly Gln Glu Arg Leu Cys Leu
   50 55 60
   Ala Gln Ile Ser Asn Thr Leu Leu Lys Asn Phe Ser Tyr Asn Glu Ile
   65 70 75 80
   His Asn Arg Arg Val Ala Leu Gly Ile Thr Cys Val Gln Cys Thr Pro
   85 90 95
   Val Gln Leu Glu Ile Leu Arg Arg Ala Gly Ala Met Pro Ile Ser Ser
   100 105 110
   Arg Arg Cys Gly Met Ile Thr Lys Arg Glu Ala Glu Arg Leu Cys Lys
   115 120 125
   Ser Phe Leu Gly Glu Asn Arg Pro Pro Lys Leu Pro Asp Asn Phe Ala
   130 135 140
   Phe Asp Val Ser His Glu Cys Ala Trp Gly Cys Arg Gly Ser Phe Ile
   145 150 155 160
   Pro Ala Arg Tyr Asn Ser Ser Arg Ala Lys Cys Ile Lys Cys Ser Tyr
   165 170 175
   Cys Asn Met Tyr Phe Ser Pro Asn Lys Phe Ile Phe His Ser His Arg
   180 185 190
   Thr Pro Asp Ala Lys Tyr Thr Gln Pro Asp Ala Ala Asn Phe Asn Ser
   195 200 205
   Trp Arg Arg His Leu Lys Leu Thr Asp Lys Ser Pro Gln Asp Glu Leu
   210 215 220
   Val Phe Ala Trp Glu Asp Val Lys Ala Met Phe Asn Gly Gly Ser Arg
   225 230 235 240
   Lys Arg Ala Leu Pro Gln Pro Ser Ala His Pro Ala Cys His Pro Leu
   245 250 255
   Ser Ser Val Lys Ala Ala Ala Val Ala Ala Ala Ala Ala Val Ala Gly
   260 265 270
   Gly Gly Gly Leu Leu Gly Pro His Leu Leu Gly Ala Pro Pro Pro Pro
   275 280 285
   Pro Pro Pro Pro Pro Leu Ala Glu Leu Ala Gly Ala Pro His Ala His
   290 295 300
   His Lys Arg Pro Arg Phe Asp Asp Asp Asp Asp Ser Leu Gln Glu Ala
   305 310 315 320
   Ala Val Val Ala Ala Ala Ser Leu Ser Ala Ala Ala Ala Ser Leu Ser
   325 330 335
   Val Ala Ala Ala Thr Gly Gly Ala Gly Pro Gly Ala Gly Gly Pro Gly
   340 345 350
   Gly Gly Cys Val Ala Gly Val Gly Val Gly Ala Ser Ala Gly Ala Gly
   355 360 365
   Ala Ala Ala Gly Thr Lys Gly Pro Arg Ser Tyr Pro Val Ile Pro Val
   370 375 380
   Pro Ser Lys Gly Ser Phe Gly Gly Val Leu Gln Lys Phe Pro Gly Cys
   385 390 395 400
   Gly Gly Leu Phe Pro His Pro Tyr Thr Phe Pro Ala Ala Ala Ala Ala
   405 410 415
   Phe Gly Leu Cys His Lys Lys Glu Asp Ala Gly Thr Ala Ala Glu Ala
   420 425 430
   Leu Gly Gly Ala Gly Ala Gly Ser Ala Gly Ala Ala Pro Lys Ala Gly
   435 440 445
   Leu Ser Gly Leu Phe Trp Pro Ala Gly Arg Lys Asp Ala Phe Tyr Pro
   450 455 460
   Pro Phe Cys Met Phe Trp Pro Pro Arg Thr Pro Gly Gly Leu Pro Val
   465 470 475 480
   Pro Thr Tyr Leu Gln Pro Pro Pro Gln Pro Pro Ser Ala Leu Gly Cys
   485 490 495
   Ala Leu Gly Asp Ser Pro Ala Leu Leu Arg Gln Ala Phe Leu Asp Leu
   500 505 510
   Ala Glu Pro Gly Gly Ala Gly Gly Ser Ala Glu Ala Ala Pro Pro Pro
   515 520 525
   Gly Gln Pro Pro Pro Val Val Ala Asn Gly Pro Gly Ser Gly Pro Pro
   530 535 540
   Ala Thr Gly Gly Thr Gly Ala Arg Asp Thr Leu Phe Glu Ser Pro Pro
   545 550 555 560
   Gly Gly Ser Gly Gly Asp Cys Ser Ala Gly Ser Thr Pro Pro Ala Glu
   565 570 575
   Gln Gly Val Thr Ser Gly Thr Gly Ser Ala Ser Ser Gly Ala Gly Ser
   580 585 590
   Val Gly Thr Arg Val Pro Ala Pro His His Pro His Leu Leu Glu Gly
   595 600 605
   Arg Lys Ala Gly Gly Gly Ser Tyr His His Ser Ser Ala Phe Arg Pro
   610 615 620
   Val Gly Gly Lys Asp Asp Ala Glu Ser Leu Ala Lys Leu His Gly Ala
   625 630 635 640
   Ser Ala Gly Thr Pro His Ser Ala Pro Ala His His His His His His
   645 650 655
   His His Pro His His His His His His Pro Pro Gln Pro Pro Ser Pro
   660 665 670
   Leu Leu Leu Leu Gln Pro Gln Pro Asp Glu Pro Gly Ser Glu Arg His
   675 680 685
   His Pro Ala Pro Pro Pro Pro Pro Pro Pro Pro Pro Leu Ala Pro Gln
   690 695 700
   Pro His His Arg Gly Leu Leu Ser Pro Glu Gly Thr Ser Cys Ser Tyr
   705 710 715 720
   Pro Ser Glu Asp Ser Ser Glu Asp Glu Glu Asp Glu Glu Glu Glu Gln
   725 730 735
   Glu Val Asp Val Glu Gly His Lys Pro Leu Glu Gly Glu Glu Glu Glu
   740 745 750
   Asp Gly Arg Asp Pro Glu Asp Glu Glu Glu Glu Asp Glu Glu Thr Arg
   755 760 765
   Val Leu Leu Gly Asp Ser Leu Val Gly Gly Gly Arg Phe Leu Gln Gly
   770 775 780
   Arg Gly Leu Ser Glu Lys Gly Ser Gly Arg Asp Arg Thr Thr Pro Ala
   785 790 795 800
   Val Gly Ala Phe Pro Leu Ala Leu Asn Ser Ser Arg Leu Leu Gln Glu
   805 810 815
   Asp Gly Lys Leu Gly Asp Ser Gly Gly Ser Asp Leu Pro Ala Pro Pro
   820 825 830
   Pro Pro Pro Leu Ala Pro Gln Lys Ala Ser Ser Ser Gly Gly Ser Arg
   835 840 845
   Pro Gly Ser Pro Val His His Pro Ser Leu Glu Glu Glu Pro Ser Tyr
   850 855 860
   Lys Asp Asn Gln Lys Pro Lys Glu Asn Asn Gln Val Ile Ile Ser Thr
   865 870 875 880
   Lys Asp Asp Asn Phe Ser Asp Lys Asn Lys Gly His Gly Phe Phe Ile
   885 890 895
   Thr Asp Ser Asp Ser Ser Gly Asp Phe Trp Arg Glu Arg Ser Gly Glu
   900 905 910
   His Thr Gln Glu Thr Asn Ser Pro His Ser Leu Lys Lys Asp Val Glu
   915 920 925
   Asn Met Gly Lys Glu Glu Leu Gln Lys Val Leu Phe Glu Gln Ile Asp
   930 935 940
   Leu Arg Arg Arg Leu Glu Gln Glu Phe Gln Val Leu Lys Gly Asn Thr
   945 950 955 960
   Ser Phe Pro Val Phe Asn Asn Phe Gln Asp Gln Met Lys Arg Glu Leu
   965 970 975
   Ala Tyr Arg Glu Glu Met Val Gln Gln Leu Gln Ile Ile Pro Tyr Ala
   980 985 990
   Ala Ser Leu Ile Arg Lys Glu Lys Leu Gly Ala His Leu Ser Lys Ser
   995 1000 1005
<210> 2
   <211> 4156
   <212> DNA
   <213> Mus musculus
<220>
   <221> CDS
   <222> (357)..(3383)
<400> 2
   gagttcagag acagagaaag gcgctgtcag actacgctcc acttggggct cgcttgccgc    60
   gggtttccgc gcacccacac tttctgtggt ggtggggaca ccctgcccca atccaggccc   120
   ccattctgcc caccaccccc ccgccgggat ttgagcgcca ttagctcgcg ccagtaccgg   180
   gatcttcgct cagctcgcct gagtcccgcc cgctggattc gaggacccgc ttggatgctt   240
   cgcctccgag cgccctcgga agatgggccg gcagctacaa gcctaaagac gtcaagggct   300
   taccagtctc caaggcaaac ccggagctct aagcccgctg ttcttccgtg gaggag atg   359
                                                                 Met
                                                                 1
   gct tcc agc cca ctg cca ggg ccc aat gat atc cta ctt gca tcg cca    407
   Ala Ser Ser Pro Leu Pro Gly Pro Asn Asp Ile Leu Leu Ala Ser Pro
               5                   10                  15
   tcg agc gcc ttc cag ccc gac gca ttg agc caa ccg cgg ccg ggt cac    455
   Ser Ser Ala Phe Gln Pro Asp Ala Leu Ser Gln Pro Arg Pro Gly His
           20                  25                  30
   gcc aac ctt aaa ccc aac cag gtg ggc cag gtg atc ctc tat ggc att    503
   Ala Asn Leu Lys Pro Asn Gln Val Gly Gln Val Ile Leu Tyr Gly Ile
       35                  40                  45
   ccc atc gtg tcc ttg gtg atc gac ggg cag gag cgc ctg tgc ctg gcg    551
   Pro Ile Val Ser Leu Val Ile Asp Gly Gln Glu Arg Leu Cys Leu Ala
   50                  55                  60                  65
   cag atc tcc aac act ctt ctc aag aac ttc agc tat aac gag atc cac    599
   Gln Ile Ser Asn Thr Leu Leu Lys Asn Phe Ser Tyr Asn Glu Ile His
                   70                  75                  80
   aac cgc cga gtg gct ctg ggc atc acg tgc gtg cag tgc aca cct gtc    647
   Asn Arg Arg Val Ala Leu Gly Ile Thr Cys Val Gln Cys Thr Pro Val
   85 90 95
   caa ctg gag atc ctg cgg cgg gcc ggg gcc atg ccc atc tct tcg cgg 695
   Gln Leu Glu Ile Leu Arg Arg Ala Gly Ala Met Pro Ile Ser Ser Arg
   100 105 110
   cgt tgt ggc atg atc acc aag cgt gag gcc gag cgc ctt tgt aag tca 743
   Arg Cys Gly Met Ile Thr Lys Arg Glu Ala Glu Arg Leu Cys Lys Ser
   115 120 125
   ttc ctg ggc gaa aat cgg cca ccc aag ctg ccg gac aac ttc gcc ttc 791
   Phe Leu Gly Glu Asn Arg Pro Pro Lys Leu Pro Asp Asn Phe Ala Phe
   130 135 140 145
   gac gtg tcg cac gag tgt gcc tgg ggc tgc cgc ggc agc ttc atc ccg 839
   Asp Val Ser His Glu Cys Ala Trp Gly Cys Arg Gly Ser Phe Ile Pro
   150 155 160
   gcg cgc tac aac agc tct cgc gcc aag tgc atc aag tgt agc tac tgc 887
   Ala Arg Tyr Asn Ser Ser Arg Ala Lys Cys Ile Lys Cys Ser Tyr Cys
   165 170 175
   aac atg tac ttc tcc ccc aac aag ttc att ttc cac tcc cac cgc acc 935
   Asn Met Tyr Phe Ser Pro Asn Lys Phe Ile Phe His Ser His Arg Thr
   180 185 190
   cca gac gcc aag tac aca cag cca gac gcg gct aac ttc aat tcc tgg 983
   Pro Asp Ala Lys Tyr Thr Gln Pro Asp Ala Ala Asn Phe Asn Ser Trp
   195 200 205
   cgc cgc cat ctc aag ctc aca gac aag agc cct cag gac gag cta gtc 1031
   Arg Arg His Leu Lys Leu Thr Asp Lys Ser Pro Gln Asp Glu Leu Val
   210 215 220 225
   ttc gcc tgg gag gac gtc aag gcc atg ttc aac ggt ggc agc cgc aag 1079
   Phe Ala Trp Glu Asp Val Lys Ala Met Phe Asn Gly Gly Ser Arg Lys
   230 235 240
   cgc gcg cta cct caa ccc agc gcg cac ccg gcc tgt cac cca ctc agt 1127
   Arg Ala Leu Pro Gln Pro Ser Ala His Pro Ala Cys His Pro Leu Ser
   245 250 255
   tcc gtc aag gct gct gcg gtg gcg gcc gca gcc gca gtg gcc ggg ggc 1175
   Ser Val Lys Ala Ala Ala Val Ala Ala Ala Ala Ala Val Ala Gly Gly
   260 265 270
   gga ggc ctg ctg ggc ccg cac ctg ctg ggg gct cca ccc ccg cca ccg 1223
   Gly Gly Leu Leu Gly Pro His Leu Leu Gly Ala Pro Pro Pro Pro Pro
   275 280 285
   ccg cca cca ccc ttg gct gag ctg gcg ggc gca cct cac gcc cat cac 1271
   Pro Pro Pro Pro Leu Ala Glu Leu Ala Gly Ala Pro His Ala His His
   290 295 300 305
   aag cgg ccg cgc ttc gac gac gat gac gat tcc ctc cag gaa gct gcc 1319
   Lys Arg Pro Arg Phe Asp Asp Asp Asp Asp Ser Leu Gln Glu Ala Ala
   310 315 320
   gtg gtg gct gca gcc agc ctc tcg gca gcc gca gcc agc ctc tcg gtg 1367
   Val Val Ala Ala Ala Ser Leu Ser Ala Ala Ala Ala Ser Leu Ser Val
   325 330 335
   gcc gca gcc aca ggc ggc gcc ggg cca ggc gca ggt ggc ccc ggg ggt 1415
   Ala Ala Ala Thr Gly Gly Ala Gly Pro Gly Ala Gly Gly Pro Gly Gly
   340 345 350
   ggc tgc gtg gcc ggc gtg ggc gtg ggt gcc agt gcg ggg gct ggt gca 1463
   Gly Cys Val Ala Gly Val Gly Val Gly Ala Ser Ala Gly Ala Gly Ala
   355 360 365
   gca gct ggc acc aaa ggt cca cgc agc tac cca gtc atc cca gtg ccc 1511
   Ala Ala Gly Thr Lys Gly Pro Arg Ser Tyr Pro Val Ile Pro Val Pro
   370 375 380 385
   agc aag ggt tcg ttc ggg ggc gtg cta cag aag ttc ccg ggc tgc ggg      1559
   Ser Lys Gly Ser Phe Gly Gly Val Leu Gln Lys Phe Pro Gly Cys Gly
                   390                 395                 400
   ggc ctc ttc ccg cat cct tac acc ttc ccg gca gcg gcc gca gcc ttc      1607
   Gly Leu Phe Pro His Pro Tyr Thr Phe Pro Ala Ala Ala Ala Ala Phe
               405                 410                 415
   ggc ttg tgc cac aag aag gag gac gcg ggg aca gcg gcc gag gcc ctg      1655
   Gly Leu Cys His Lys Lys Glu Asp Ala Gly Thr Ala Ala Glu Ala Leu
           420                 425                 430
   ggg gga gcg ggc gcg ggg agc gca ggt gcg gcg ccc aag gca ggg ctg      1703
   Gly Gly Ala Gly Ala Gly Ser Ala Gly Ala Ala Pro Lys Ala Gly Leu
       435                 440                 445
   tcg ggt ctc ttc tgg ccc gcg ggt cgc aag gac gcc ttc tac cct ccc      1751
   Ser Gly Leu Phe Trp Pro Ala Gly Arg Lys Asp Ala Phe Tyr Pro Pro
   450                 455                 460                 465
   ttc tgc atg ttc tgg cca ccg cgg acc ccc ggc ggg ctg ccc gtg ccc      1799
   Phe Cys Met Phe Trp Pro Pro Arg Thr Pro Gly Gly Leu Pro Val Pro
                   470                 475                 480
   acc tac cta cag ccc ccg ccg cag ccc ccg tct gcg ctc ggc tgc gcg      1847
   Thr Tyr Leu Gln Pro Pro Pro Gln Pro Pro Ser Ala Leu Gly Cys Ala
               485                 490                 495
   ctg ggt gat agc ccg gcc ctg ctg cgt cag gcc ttc ctg gac ctg gcc      1895
   Leu Gly Asp Ser Pro Ala Leu Leu Arg Gln Ala Phe Leu Asp Leu Ala
           500                 505                 510
   gag ccg ggc ggt gca ggt ggc agc gcc gag gca gcg ccc cct ccg ggc      1943
   Glu Pro Gly Gly Ala Gly Gly Ser Ala Glu Ala Ala Pro Pro Pro Gly
       515                 520                 525
   caa cct ccc ccc gtg gtg gcc aat ggc cct ggc tcc ggt cct cca gct 1991
   Gln Pro Pro Pro Val Val Ala Asn Gly Pro Gly Ser Gly Pro Pro Ala
   530 535 540 545
   act ggg ggc act gga gca cgc gac acg ctc ttc gag tcg ccc ccg ggc 2039
   Thr Gly Gly Thr Gly Ala Arg Asp Thr Leu Phe Glu Ser Pro Pro Gly
   550 555 560
   ggc agc ggc ggg gac tgc agc gcc ggg tcc acg cca ccc gca gag caa 2087
   Gly Ser Gly Gly Asp Cys Ser Ala Gly Ser Thr Pro Pro Ala Glu Gln
   565 570 575
   gga gtg acg tcc ggg acc ggg tct gcg tcc tcc gga gca ggc tct gtg 2135
   Gly Val Thr Ser Gly Thr Gly Ser Ala Ser Ser Gly Ala Gly Ser Val
   580 585 590
   ggc acc cga gtg ccg gct ccc cat cac ccg cac ctc ctg gaa ggg cgc 2183
   Gly Thr Arg Val Pro Ala Pro His His Pro His Leu Leu Glu Gly Arg
   595 600 605
   aag gcg ggc ggc ggc agc tac cac cat tcc agc gcc ttc cgt ccg gtg 2231
   Lys Ala Gly Gly Gly Ser Tyr His His Ser Ser Ala Phe Arg Pro Val
   610 615 620 625
   ggc ggc aag gac gac gca gaa agc ctg gcc aag ctg cac ggg gcg tcg 2279
   Gly Gly Lys Asp Asp Ala Glu Ser Leu Ala Lys Leu His Gly Ala Ser
   630 635 640
   gcg ggc aca ccc cac tca gcc cca gcg cat cac cat cac cac cac cat 2327
   Ala Gly Thr Pro His Ser Ala Pro Ala His His His His His His His
   645 650 655
   cac ccg cac cat cac cac cat cac cct ccg cag ccg ccg tcg cca ctg 2375
   His Pro His His His His His His Pro Pro Gln Pro Pro Ser Pro Leu
   660 665 670
   ctg ctg ttg cag ccc cag ccc gat gag ccg ggg tcg gag cgc cac cac 2423
   Leu Leu Leu Gln Pro Gln Pro Asp Glu Pro Gly Ser Glu Arg His His
   675 680 685
   cca gcc ccg cca ccc ccg cca ccg ccg ccc cct ctg gcc ccg cag ccg 2471
   Pro Ala Pro Pro Pro Pro Pro Pro Pro Pro Pro Leu Ala Pro Gln Pro
   690 695 700 705
   cac cac cga ggc ctt ctg tcc ccc gag ggc acc agc tgc agc tac ccc 2519
   His His Arg Gly Leu Leu Ser Pro Glu Gly Thr Ser Cys Ser Tyr Pro
   710 715 720
   agt gag gac agc tct gaa gac gag gag gac gag gag gaa gag cag gag 2567
   Ser Glu Asp Ser Ser Glu Asp Glu Glu Asp Glu Glu Glu Glu Gln Glu
   725 730 735
   gtg gac gtg gag ggc cac aag cca ctc gaa ggc gag gaa gag gag gac 2615
   Val Asp Val Glu Gly His Lys Pro Leu Glu Gly Glu Glu Glu Glu Asp
   740 745 750
   ggt cgc gat cct gaa gat gag gag gaa gaa gat gag gag acc cgg gtc 2663
   Gly Arg Asp Pro Glu Asp Glu Glu Glu Glu Asp Glu Glu Thr Arg Val
   755 760 765
   ctt cta gga gac tcc ctg gtt ggc ggt ggc cgg ttc ctc cag ggc cga 2711
   Leu Leu Gly Asp Ser Leu Val Gly Gly Gly Arg Phe Leu Gln Gly Arg
   770 775 780 785
   ggg cta tcg gag aag ggg agc ggt cgg gac cgc acg acg ccc gcc gtg 2759
   Gly Leu Ser Glu Lys Gly Ser Gly Arg Asp Arg Thr Thr Pro Ala Val
   790 795 800
   ggt gct ttc cct cta gcg ctg aac tcc tcc agg ctg cta caa gag gat 2807
   Gly Ala Phe Pro Leu Ala Leu Asn Ser Ser Arg Leu Leu Gln Glu Asp
   805 810 815
   ggg aaa ctg ggg gac tct gga ggc tcg gac ctg ccg gcg ccc ccg ccc 2855
   Gly Lys Leu Gly Asp Ser Gly Gly Ser Asp Leu Pro Ala Pro Pro Pro
   820 825 830
   cca ccc ctg gcc ccc cag aaa gca agc agc agt ggg ggc agc agg cca 2903
   Pro Pro Leu Ala Pro Gln Lys Ala Ser Ser Ser Gly Gly Ser Arg Pro
   835 840 845
   ggc agc cct gtc cac cat cca tca ctg gag gag gag ccc tcg tac aaa 2951
   Gly Ser Pro Val His His Pro Ser Leu Glu Glu Glu Pro Ser Tyr Lys
   850 855 860 865
   gat aat cag aaa cct aag gaa aac aac caa gtt att ata tct aca aag 2999
   Asp Asn Gln Lys Pro Lys Glu Asn Asn Gln Val Ile Ile Ser Thr Lys
   870 875 880
   gat gac aac ttc tca gat aag aac aag gga cat ggc ttc ttc atc aca 3047
   Asp Asp Asn Phe Ser Asp Lys Asn Lys Gly His Gly Phe Phe Ile Thr
   885 890 895
   gat tct gat tct tct gga gac ttt tgg aga gaa aga tca ggt gaa cat 3095
   Asp Ser Asp Ser Ser Gly Asp Phe Trp Arg Glu Arg Ser Gly Glu His
   900 905 910
   aca caa gaa acc aat tca cct cat tcg ctc aaa aag gat gta gaa aac 3143
   Thr Gln Glu Thr Asn Ser Pro His Ser Leu Lys Lys Asp Val Glu Asn
   915 920 925
   atg gga aaa gag gaa ctt cag aag gtt ttg ttt gag caa ata gat ttg 3191
   Met Gly Lys Glu Glu Leu Gln Lys Val Leu Phe Glu Gln Ile Asp Leu
   930 935 940 945
   cgg agg cgg ctg gag caa gaa ttc caa gtg tta aaa gga aat acg tcc 3239
   Arg Arg Arg Leu Glu Gln Glu Phe Gln Val Leu Lys Gly Asn Thr Ser
   950 955 960
   ttc cca gtc ttc aat aac ttt cag gat cag atg aaa agg gaa ctg gct 3287
   Phe Pro Val Phe Asn Asn Phe Gln Asp Gln Met Lys Arg Glu Leu Ala
   965 970 975
   tac cga gaa gag atg gtg caa cag tta caa atc atc ccc tat gca gca 3335
   Tyr Arg Glu Glu Met Val Gln Gln Leu Gln Ile Ile Pro Tyr Ala Ala
   980 985 990
   agc ttg atc agg aaa gag aag ctt ggc gcc cat ctc agc aaa agc taa 3383
   Ser Leu Ile Arg Lys Glu Lys Leu Gly Ala His Leu Ser Lys Ser
   995 1000 1005
   aaggcgacag acccactcac tcttgttctg taagatacag cctaccactg agcacttcgg 3443
   acctgcagaa agaagaactg caaactgaaa gggcttgggc accaaaacca ggttcgagag 3503
   aagccaagga caagtgacct cgcgccagca tgggcaacca tgtaaaatag actgtggcgg 3563
   ccattcatta ggaggggggg gaggggggag ccaaccagag cggtcaatgc ttgtcacatc 3623
   ttttggtgga accaaagttt gaatatttgt gttttgaaag catagctgat cccagagggt 3683
   aggaagtgct gagtggggaa atgtttgtgt ggtctctttg gcgccatacg attatccttt 3743
   gctcttccgg aagtaattca tagtggaaag aggacagaat ggggacttag gtttagacaa 3803
   acctgctttc cataccaagc tggacagaca cacatggccc cttcctctct ggtactttgc 3863
   ctgctgtatg aagattgtat tcctctggaa atattttaca gtttaatatt gagtgtaatt 3923
   aagaatataa tcatgttatc aaaaatggta tttaactctg ttgtagtttc tttaacattc 3983
   atgtggataa aaagtttata ataaaaaaac tatgacgtaa tagatgtgtt catgtagtta 4043
   agtgcatata tgcttggggg caactcagaa acgtaatgct ttttagagtt attttggcat 4103
   aaagtatttg aatataatta tttttgaaaa caaaaaaaaa aaaaaaaaaa aaa 4156
<210> 3
   <211> 1014
   <212> PRT
   <213> Homo sapiens
<400> 3
   Met Ala Ser Ser Pro Leu Pro Gly Pro Asn Asp Ile Leu Leu Ala Ser
   1               5                   10                  15
   Pro Ser Ser Ala Phe Gln Pro Asp Thr Leu Ser Gln Pro Arg Pro Gly
               20                  25                  30
   His Ala Asn Leu Lys Pro Asn Gln Val Gly Gln Val Ile Leu Tyr Gly
           35                  40                  45
   Ile Pro Ile Val Ser Leu Val Ile Asp Gly Gln Glu Arg Leu Cys Leu
       50                  55                  60
   Ala Gln Ile Ser Asn Thr Leu Leu Lys Asn Phe Ser Tyr Asn Glu Ile
   65                  70                  75                  80
   His Asn Arg Arg Val Ala Leu Gly Ile Thr Cys Val Gln Cys Thr Pro
                   85                  90                  95
   Val Gln Leu Glu Ile Leu Arg Arg Ala Gly Ala Met Pro Ile Ser Ser
               100                 105                 110
   Arg Arg Cys Gly Met Ile Thr Lys Arg Glu Ala Glu Arg Leu Cys Lys
           115                 120                 125
   Ser Phe Leu Gly Glu Asn Arg Pro Pro Lys Leu Pro Asp Asn Phe Ala
       130                 135                 140
   Phe Asp Val Ser His Glu Cys Ala Trp Gly Cys Arg Gly Ser Phe Ile
   145                 150                 155                 160
   Pro Ala Arg Tyr Asn Ser Ser Arg Ala Lys Cys Ile Lys Cys Ser Tyr
                   165                 170                 175
   Cys Asn Met Tyr Phe Ser Pro Asn Lys Phe Ile Phe His Ser His Arg
   180 185 190
   Thr Pro Asp Ala Lys Tyr Thr Gln Pro Asp Ala Ala Asn Phe Asn Ser
   195 200 205
   Trp Arg Arg His Leu Lys Leu Thr Asp Lys Ser Pro Gln Asp Glu Leu
   210 215 220
   Val Phe Ala Trp Glu Asp Val Lys Ala Met Phe Asn Gly Gly Ser Arg
   225 230 235 240
   Lys Arg Ala Leu Pro Gln Pro Gly Ala His Pro Ala Cys His Pro Leu
   245 250 255
   Ser Ser Val Lys Ala Ala Ala Val Ala Ala Ala Ala Ala Val Ala Gly
   260 265 270
   Gly Gly Gly Leu Leu Gly Pro His Leu Leu Gly Ala Pro Pro Pro Pro
   275 280 285
   Pro Pro Pro Pro Pro Pro Leu Ala Glu Leu Ala Gly Ala Pro His Ala
   290 295 300
   His His Lys Arg Pro Arg Phe Asp Asp Asp Asp Asp Ser Leu Gln Glu
   305 310 315 320
   Ala Ala Val Val Ala Ala Ala Ser Leu Ser Ala Ala Ala Ala Ser Leu
   325 330 335
   Ser Val Ala Ala Ala Ser Gly Gly Ala Gly Thr Gly Gly Gly Gly Ala
   340 345 350
   Gly Gly Gly Cys Val Ala Gly Val Gly Val Gly Ala Gly Ala Gly Ala
   355 360 365
   Gly Ala Gly Ala Gly Ala Lys Gly Pro Arg Ser Tyr Pro Val Ile Pro
   370 375 380
   Val Pro Ser Lys Gly Ser Phe Gly Gly Val Leu Gln Lys Phe Pro Gly
   385 390 395 400
   Cys Gly Gly Leu Phe Pro His Pro Tyr Thr Phe Pro Ala Ala Ala Ala
   405 410 415
   Ala Phe Ser Leu Cys His Lys Lys Glu Asp Ala Gly Ala Ala Ala Glu
   420 425 430
   Ala Leu Gly Gly Ala Gly Ala Gly Gly Ala Gly Ala Ala Pro Lys Ala
   435 440 445
   Gly Leu Ser Gly Leu Phe Trp Pro Ala Gly Arg Lys Asp Ala Phe Tyr
   450 455 460
   Pro Pro Phe Cys Met Phe Trp Pro Pro Arg Thr Pro Gly Gly Leu Pro
   465 470 475 480
   Val Pro Thr Tyr Leu Gln Pro Pro Pro Gln Pro Pro Ser Ala Leu Gly
   485 490 495
   Cys Ala Leu Gly Glu Ser Pro Ala Leu Leu Arg Gln Ala Phe Leu Asp
   500 505 510
   Leu Ala Glu Pro Gly Gly Ala Ala Gly Ser Ala Glu Ala Ala Pro Pro
   515 520 525
   Pro Gly Gln Pro Pro Gln Val Val Ala Asn Gly Pro Gly Ser Gly Pro
   530 535 540
   Pro Pro Pro Ala Gly Gly Ala Gly Ser Arg Asp Ala Leu Phe Glu Ser
   545 550 555 560
   Pro Pro Gly Gly Ser Gly Gly Asp Cys Ser Ala Gly Ser Thr Pro Pro
   565 570 575
   Ala Asp Ser Val Ala Ala Ala Gly Ala Gly Ala Ala Ala Ala Gly Ser
   580 585 590
   Gly Pro Ala Gly Ser Arg Val Pro Ala Pro His His Pro His Leu Leu
   595 600 605
   Glu Gly Arg Lys Ala Gly Gly Gly Ser Tyr His His Ser Ser Ala Phe
   610 615 620
   Arg Pro Val Gly Gly Lys Asp Asp Ala Glu Ser Leu Ala Lys Leu His
   625 630 635 640
   Gly Ala Ser Ala Gly Ala Pro His Ser Ala Gln Thr His Pro His His
   645 650 655
   His His His Pro His His His His His His His His Pro Pro Gln Pro
   660 665 670
   Pro Ser Pro Leu Leu Leu Leu Pro Pro Gln Pro Asp Glu Pro Gly Ser
   675 680 685
   Glu Arg His His Pro Ala Pro Pro Pro Pro Pro Pro Pro Pro Pro Pro
   690 695 700
   Pro Pro Leu Ala Gln His Pro His His Arg Gly Leu Leu Ser Pro Gly
   705 710 715 720
   Gly Thr Ser Cys Cys Tyr Pro Ser Glu Asp Ser Ser Glu Asp Glu Asp
   725 730 735
   Asp Glu Glu Glu Glu Gln Glu Val Asp Val Glu Gly His Lys Pro Pro
   740 745 750
   Glu Gly Glu Glu Glu Glu Glu Gly Arg Asp Pro Asp Asp Asp Glu Glu
   755 760 765
   Glu Asp Glu Glu Thr Glu Val Leu Leu Gly Asp Pro Leu Val Gly Gly
   770 775 780
   Gly Arg Phe Leu Gln Gly Arg Gly Pro Ser Glu Lys Gly Ser Ser Arg
   785 790 795 800
   Asp Arg Ala Pro Ala Val Ala Gly Ala Phe Pro Leu Gly Leu Asn Ser
   805 810 815
   Ser Arg Leu Leu Gln Glu Asp Gly Lys Leu Gly Asp Pro Gly Ser Asp
   820 825 830
   Leu Pro Pro Pro Pro Pro Pro Pro Leu Ala Pro Gln Lys Ala Ser Gly
   835 840 845
   Gly Gly Ser Ser Ser Pro Gly Ser Pro Val His His Pro Ser Leu Glu
   850 855 860
   Glu Gln Pro Ser Tyr Lys Asp Ser Gln Lys Thr Lys Glu Asn Asn Gln
   865 870 875 880
   Val Ile Val Ser Thr Lys Asp Asp Asn Val Leu Asp Lys Asn Lys Glu
   885 890 895
   His Ser Phe Phe Ile Thr Asp Ser Asp Ala Ser Gly Gly Asp Phe Trp
   900 905 910
   Arg Glu Arg Ser Gly Glu His Thr Gln Glu Thr Asn Ser Pro His Ser
   915 920 925
   Leu Lys Lys Asp Val Glu Asn Met Gly Lys Glu Glu Leu Gln Lys Val
   930 935 940
   Leu Phe Glu Gln Ile Asp Leu Arg Arg Arg Leu Glu Gln Glu Phe Gln
   945 950 955 960
   Val Leu Lys Gly Asn Thr Ser Phe Pro Val Phe Asn Asn Phe Gln Asp
   965 970 975
   Gln Met Lys Arg Glu Leu Ala Tyr Arg Glu Glu Met Val Gln Gln Leu
   980 985 990
   Gln Ile Ile Pro Tyr Ala Ala Ser Leu Ile Arg Lys Glu Lys Leu Gly
   995 1000 1005
   Ala His Leu Ser Lys Ser
   1010
<210> 4
   <211> 3045
   <212> DNA
   <213> Homo sapiens
<220>
   <221> CDS
   <222> (1).. (3045)
<400> 4
   atg gct tcc agt ccg ctg cca ggg ccc aac gac atc ctg ctg gcg tcg 48
   Met Ala Ser Ser Pro Leu Pro Gly Pro Asn Asp Ile Leu Leu Ala Ser
   1 5 10 15
   ccg tcg agc gcc ttc cag ccc gac acg ctg agc cag ccg cgg cca ggg 96
   Pro Ser Ser Ala Phe Gln Pro Asp Thr Leu Ser Gln Pro Arg Pro Gly
   20 25 30
   cac gcc aac ctc aaa ccc aac cag gtg ggc cag gtg atc ctc tac ggc 144
   His Ala Asn Leu Lys Pro Asn Gln Val Gly Gln Val Ile Leu Tyr Gly
   35 40 45
   att ccc atc gtg tcg ttg gtg atc gac ggg caa gag cgc ctg tgc ctg 192
   Ile Pro Ile Val Ser Leu Val Ile Asp Gly Gln Glu Arg Leu Cys Leu
   50 55 60
   gcg cag atc tcc aac act ctg ctc aag aac ttc agc tac aac gag atc 240
   Ala Gln Ile Ser Asn Thr Leu Leu Lys Asn Phe Ser Tyr Asn Glu Ile
   65 70 75 80
   cac aac cgt cgc gtg gca ctg ggc atc acg tgt gtg cag tgc acg ccg 288
   His Asn Arg Arg Val Ala Leu Gly Ile Thr Cys Val Gln Cys Thr Pro
   85 90 95
   gtg caa ctg gag atc ctg cgg cgt gcc ggg gcc atg ccc atc tca tcg 336
   Val Gln Leu Glu Ile Leu Arg Arg Ala Gly Ala Met Pro Ile Ser Ser
   100 105 110
   cgc cgc tgc ggc atg atc acc aaa cgc gag gcc gag cgt ctg tgc aag 384
   Arg Arg Cys Gly Met Ile Thr Lys Arg Glu Ala Glu Arg Leu Cys Lys
   115 120 125
   tcg ttc ctg ggc gaa aac agg ccg ccc aag ctg cca gac aat ttc gcc 432
   Ser Phe Leu Gly Glu Asn Arg Pro Pro Lys Leu Pro Asp Asn Phe Ala
   130 135 140
   ttc gac gtg tca cac gag tgc gcc tgg ggc tgc cgc ggc agc ttc att 480
   Phe Asp Val Ser His Glu Cys Ala Trp Gly Cys Arg Gly Ser Phe Ile
   145 150 155 160
   ccc gcg cgc tac aac agc tcg cgc gcc aag tgc atc aaa tgc agc tac 528
   Pro Ala Arg Tyr Asn Ser Ser Arg Ala Lys Cys Ile Lys Cys Ser Tyr
   165 170 175
   tgc aac atg tac ttc tcg ccc aac aag ttc att ttc cac tcc cac cgc 576
   Cys Asn Met Tyr Phe Ser Pro Asn Lys Phe Ile Phe His Ser His Arg
   180 185 190
   acg ccc gac gcc aag tac act cag cca gac gca gcc aac ttc aac tcg 624
   Thr Pro Asp Ala Lys Tyr Thr Gln Pro Asp Ala Ala Asn Phe Asn Ser
   195 200 205
   tgg cgc cgt cat ctc aag ctc acc gac aag agt ccc cag gac gag ctg 672
   Trp Arg Arg His Leu Lys Leu Thr Asp Lys Ser Pro Gln Asp Glu Leu
   210 215 220
   gtc ttc gcc tgg gag gac gtc aag gcc atg ttc aac ggc ggc agc cgc 720
   Val Phe Ala Trp Glu Asp Val Lys Ala Met Phe Asn Gly Gly Ser Arg
   225 230 235 240
   aag cgc gca ctg ccc cag ccg ggc gcg cac ccc gcc tgc cac ccg ctc 768
   Lys Arg Ala Leu Pro Gln Pro Gly Ala His Pro Ala Cys His Pro Leu
   245 250 255
   agc tct gtg aag gcg gcc gcc gtg gcc gcc gcg gcc gcg gtg gcc gga 816
   Ser Ser Val Lys Ala Ala Ala Val Ala Ala Ala Ala Ala Val Ala Gly
   260 265 270
   ggc ggg ggt ctg ctg ggc ccc cac ctg ctg ggt gcg ccc ccg ccg ccg 864
   Gly Gly Gly Leu Leu Gly Pro His Leu Leu Gly Ala Pro Pro Pro Pro
   275 280 285
   ccg ccg cca ccg ccg ccc ttg gca gag ctg gct ggt gcc ccg cac gcc 912
   Pro Pro Pro Pro Pro Pro Leu Ala Glu Leu Ala Gly Ala Pro His Ala
   290 295 300
   cat cac aag cgg ccg cgc ttc gac gac gac gac gac tcc ttg cag gag 960
   His His Lys Arg Pro Arg Phe Asp Asp Asp Asp Asp Ser Leu Gln Glu
   305 310 315 320
   gcc gcc gta gtg gcc gcc gcc agc ctc tcg gcc gca gcc gcc agc ctc 1008
   Ala Ala Val Val Ala Ala Ala Ser Leu Ser Ala Ala Ala Ala Ser Leu
   325 330 335
   tct gtg gct gct gct tcg ggc ggc gcg ggg act ggt ggg ggc ggc gct 1056
   Ser Val Ala Ala Ala Ser Gly Gly Ala Gly Thr Gly Gly Gly Gly Ala
   340 345 350
   ggg ggt ggc tgt gtg gcc ggc gtg ggc gtg ggc gcg ggc gcg ggg gcg 1104
   Gly Gly Gly Cys Val Ala Gly Val Gly Val Gly Ala Gly Ala Gly Ala
   355 360 365
   ggt gcc ggg gca ggg gcc aaa ggc ccg cgc agc tac cca gtc atc ccg 1152
   Gly Ala Gly Ala Gly Ala Lys Gly Pro Arg Ser Tyr Pro Val Ile Pro
   370 375 380
   gtg ccc agc aag ggc tcg ttc ggg ggc gtc ctg cag aag ttc ccg ggc 1200
   Val Pro Ser Lys Gly Ser Phe Gly Gly Val Leu Gln Lys Phe Pro Gly
   385 390 395 400
   tgc ggc ggg ctc ttc ccg cac ccc tac acc ttc cct gcc gcg gcc gcc 1248
   Cys Gly Gly Leu Phe Pro His Pro Tyr Thr Phe Pro Ala Ala Ala Ala
   405 410 415
   gcc ttc agc ttg tgc cat aag aaa gag gat gcg ggt gcc gcc gct gag 1296
   Ala Phe Ser Leu Cys His Lys Lys Glu Asp Ala Gly Ala Ala Ala Glu
   420 425 430
   gcc ctg ggg ggc gcg ggc gca ggc ggc gcg ggc gcg gcg ccc aag gcc 1344
   Ala Leu Gly Gly Ala Gly Ala Gly Gly Ala Gly Ala Ala Pro Lys Ala
   435 440 445
   ggc ttg tcc ggc ctc ttc tgg ccc gcg ggc cgc aag gac gcc ttc tat 1392
   Gly Leu Ser Gly Leu Phe Trp Pro Ala Gly Arg Lys Asp Ala Phe Tyr
   450 455 460
   ccg ccc ttc tgc atg ttc tgg ccg ccg cgg acc cct ggc ggg ctc ccg 1440
   Pro Pro Phe Cys Met Phe Trp Pro Pro Arg Thr Pro Gly Gly Leu Pro
   465 470 475 480
   gtg ccc acc tac ctg cag ccc ccg cct cag ccg ccc tcg gcg cta ggc 1488
   Val Pro Thr Tyr Leu Gln Pro Pro Pro Gln Pro Pro Ser Ala Leu Gly
   485 490 495
   tgc gcg cta ggc gaa agc ccg gcc ctg ctg cgc cag gcc ttc ctg gac 1536
   Cys Ala Leu Gly Glu Ser Pro Ala Leu Leu Arg Gln Ala Phe Leu Asp
   500 505 510
   ctg gct gag cca ggc ggt gct gct ggg agc gcc gag gcc gcg ccc ccg 1584
   Leu Ala Glu Pro Gly Gly Ala Ala Gly Ser Ala Glu Ala Ala Pro Pro
   515 520 525
   ccg ggg cag ccc ccg cag gta gtg gcc aac ggc ccg ggc tcc ggc cca 1632
   Pro Gly Gln Pro Pro Gln Val Val Ala Asn Gly Pro Gly Ser Gly Pro
   530 535 540
   cct cct cct gcc ggg ggc gcc ggc tct cgc gac gcg ctc ttc gag tcg 1680
   Pro Pro Pro Ala Gly Gly Ala Gly Ser Arg Asp Ala Leu Phe Glu Ser
   545 550 555 560
   ccc ccg ggc ggc agc ggc ggg gac tgc agc gcg ggc tcc acg ccg ccc 1728
   Pro Pro Gly Gly Ser Gly Gly Asp Cys Ser Ala Gly Ser Thr Pro Pro
   565 570 575
   gcg gac tct gtg gca gct gcc ggg gca ggg gcc gcg gcc gcc ggg tct 1776
   Ala Asp Ser Val Ala Ala Ala Gly Ala Gly Ala Ala Ala Ala Gly Ser
   580 585 590
   ggc ccc gcg ggc tcc cgg gtt ccg gcg ccc cac cat ccg cac ctt ctg 1824
   Gly Pro Ala Gly Ser Arg Val Pro Ala Pro His His Pro His Leu Leu
   595 600 605
   gag ggg cgc aaa gcg ggc ggt ggc agc tac cac cat tcc agc gcc ttc 1872
   Glu Gly Arg Lys Ala Gly Gly Gly Ser Tyr His His Ser Ser Ala Phe
   610 615 620
   cgg cca gtg ggc ggc aag gac gac gcg gag agc ctg gcc aag ctg cac 1920
   Arg Pro Val Gly Gly Lys Asp Asp Ala Glu Ser Leu Ala Lys Leu His
   625 630 635 640
   ggg gcg tcg gcg ggc gcg ccc cac tcg gcc cag acg cat ccc cac cac 1968
   Gly Ala Ser Ala Gly Ala Pro His Ser Ala Gln Thr His Pro His His
   645 650 655
   cat cac cac cct cac cac cac cac cac cac cac cac ccc ccg cag ccg 2016
   His His His Pro His His His His His His His His Pro Pro Gln Pro
   660 665 670
   ccg tcg ccg ctt ctg ctg ctg ccc ccg cag ccc gac gag ccg ggt tcc 2064
   Pro Ser Pro Leu Leu Leu Leu Pro Pro Gln Pro Asp Glu Pro Gly Ser
   675 680 685
   gag cgc cac cac ccg gcc ccg ccg ccg ccg ccg ccg ccg ccc ccg ccg 2112
   Glu Arg His His Pro Ala Pro Pro Pro Pro Pro Pro Pro Pro Pro Pro
   690 695 700
   ccc cct ctg gcc cag cac ccg cac cac cga ggc ctt ctg tct ccc ggg 2160
   Pro Pro Leu Ala Gln His Pro His His Arg Gly Leu Leu Ser Pro Gly
   705 710 715 720
   gga acc agc tgc tgc tac ccc agc gag gac agc tcc gag gac gag gac 2208
   Gly Thr Ser Cys Cys Tyr Pro Ser Glu Asp Ser Ser Glu Asp Glu Asp
   725 730 735
   gac gag gaa gaa gag cag gag gtg gac gtg gag ggc cac aag ccc ccc 2256
   Asp Glu Glu Glu Glu Gln Glu Val Asp Val Glu Gly His Lys Pro Pro
   740 745 750
   gag ggc gag gaa gag gag gaa ggt cga gac cct gac gac gac gag gaa 2304
   Glu Gly Glu Glu Glu Glu Glu Gly Arg Asp Pro Asp Asp Asp Glu Glu
   755 760 765
   gag gac gag gag acg gag gtc cta ctc ggc gac ccc tta gtc ggg ggc 2352
   Glu Asp Glu Glu Thr Glu Val Leu Leu Gly Asp Pro Leu Val Gly Gly
   770 775 780
   ggc cgg ttc ctc cag ggc cga ggg ccg tcg gag aag ggg agc agc cgg 2400
   Gly Arg Phe Leu Gln Gly Arg Gly Pro Ser Glu Lys Gly Ser Ser Arg
   785 790 795 800
   gac cgc gcg ccg gcc gtc gcg ggc gcg ttc ccg ctc ggc ctg aac tcc 2448
   Asp Arg Ala Pro Ala Val Ala Gly Ala Phe Pro Leu Gly Leu Asn Ser
   805 810 815
   tcc agg ctg ctg cag gaa gac ggg aaa ctc ggg gac ccc ggc tcg gac 2496
   Ser Arg Leu Leu Gln Glu Asp Gly Lys Leu Gly Asp Pro Gly Ser Asp
   820 825 830
   ctg ccc ccg ccc ccg ccg ccg ccc ctg gcc ccc cag aag gcg agt ggc 2544
   Leu Pro Pro Pro Pro Pro Pro Pro Leu Ala Pro Gln Lys Ala Ser Gly
   835 840 845
   ggc ggc agc agc agc ccg ggc agc cca gtt cac cat cca tca ctg gag 2592
   Gly Gly Ser Ser Ser Pro Gly Ser Pro Val His His Pro Ser Leu Glu
   850 855 860
   gag cag ccc tcc tac aaa gat agt cag aaa act aag gaa aat aac caa 2640
   Glu Gln Pro Ser Tyr Lys Asp Ser Gln Lys Thr Lys Glu Asn Asn Gln
   865 870 875 880
   gta att gta tct aca aag gat gac aac gtt cta gat aag aac aag gag 2688
   Val Ile Val Ser Thr Lys Asp Asp Asn Val Leu Asp Lys Asn Lys Glu
   885 890 895
   cat agc ttt ttc atc aca gac tct gat gct tct gga gga gat ttt tgg 2736
   His Ser Phe Phe Ile Thr Asp Ser Asp Ala Ser Gly Gly Asp Phe Trp
   900 905 910
   aga gaa aga tca ggt gaa cat aca caa gaa acc aac tca cct cat tca 2784
   Arg Glu Arg Ser Gly Glu His Thr Gln Glu Thr Asn Ser Pro His Ser
   915 920 925
   ctg aaa aag gat gta gaa aat atg ggg aaa gaa gaa ctt cag aag gtt 2832
   Leu Lys Lys Asp Val Glu Asn Met Gly Lys Glu Glu Leu Gln Lys Val
   930 935 940
   tta ttt gaa caa ata gat tta cgg aga cga ctg gaa caa gaa ttc cag 2880
   Leu Phe Glu Gln Ile Asp Leu Arg Arg Arg Leu Glu Gln Glu Phe Gln
   945 950 955 960
   gtg tta aaa gga aac aca tct ttc cca gta ttc aat aat ttt cag gat 2928
   Val Leu Lys Gly Asn Thr Ser Phe Pro Val Phe Asn Asn Phe Gln Asp
   965 970 975
   cag atg aaa agg gag cta gcc tac cga gaa gaa atg gtg caa cag tta 2976
   Gln Met Lys Arg Glu Leu Ala Tyr Arg Glu Glu Met Val Gln Gln Leu
   980 985 990
   caa att atc ccc tat gca gca agt ttg atc agg aaa gaa aag ctt ggc 3024
   Gln Ile Ile Pro Tyr Ala Ala Ser Leu Ile Arg Lys Glu Lys Leu Gly
   995 1000 1005
   gcc cat ctc agc aaa agc taa 3045
   Ala His Leu Ser Lys Ser
   1010
<210> 5
   <211> 26
   <212> DNA
   <213> Artificial
<220>
   <223> An artificially synthesized oligonucleotide sequence
<400> 5
   cagctccaca acctacatca ttccgt 26
<210> 6
   <211> 12
   <212> DNA
   <213> Artificial
<220>
   <223> An artificially synthesized oligonucleotide sequence
<400> 6
   acggaatgat gt 12
<210> 7
   <211> 26
   <212> DNA
   <213> Artificial
<220>
   <223> An artificially synthesized oligonucleotide sequence
<400> 7
   gtccatcttc tctctgagac tctggt 26
<210> 8
   <211> 12
   <212> DNA
   <213> Artificial
<220>
   <223> An artificially synthesized oligonucleotide sequence
<400> 8
   accagagtct ca 12
<210> 9
   <211> 26
   <212> DNA
   <213> Artificial
<220>
   <223> An artificially synthesized oligonucleotide sequence
<400> 9
   ctgatgggtg tcttctgtga gtgtgt 26
<210> 10
   <211> 12
   <212> DNA
   <213> Artificial
<220>
   <223> An artificially synthesized oligonucleotide sequence
<400> 10
   acacactcac ag 12
<210> 11
   <211> 26
   <212> DNA
   <213> Artificial
<220>
   <223> An artificially synthesized oligonucleotide sequence
<400> 11
   ccagcatcga gaatcagtgt gacagt 26
<210> 12
   <211> 12
   <212> DNA
   <213> Artificial
<220>
   <223> An artificially synthesized oligonucleotide sequence
<400> 12
   actgtcacac tg 12
<210> 13
   <211> 26
   <212> DNA
   <213> Artificial
<220>
   <223> An artificially synthesized oligonucleotide sequence
<400> 13
   gtcgatgaac ttcgactgtc gatcgt 26
<210> 14
   <211> 12
   <212> DNA
   <213> Artificial
<220>
   <223> An artificially synthesized oligonucleotide sequence
<400> 14
   acgatcgaca gt 12
<210> 15
   <211> 26
   <212> DNA
   <213> Artificial
<220>
   <223> An artificially synthesized primer sequence
<400> 15
   acgtcaatgg cttaccagac tccaag 26
<210> 16
   <211> 26
   <212> DNA
   <213> Artificial
<220>
   <223> An artificially synthesized primer sequence
<400> 16
   tgttcttccg tggaggagat ggcttc 26
<210> 17
   <211> 26
   <212> DNA
   <213> Artificial
<220>
   <223> An artificially synthesized primer sequence
<400> 17
   cttgagaaga gtgttggaga tctgcg 26
<210> 18
   <211> 26
   <212> DNA
   <213> Artificial
<220>
   <223> An artificially synthesized primer sequence
<400> 18
   atgggaatgc catagaggat cacctg 26
<210> 19
   <211> 26
   <212> DNA
   <213> Artificial
<220>
   <223> An artificially synthesized primer sequence
<400> 19
   ggctttacac tttatgcttc cggctc 26
<210> 20
   <211> 26
   <212> DNA
   <213> Artificial
<220>
   <223> An artificially synthesized primer sequence
<400> 20
   cagctatgac catgattacg ccaagc 26
<210> 21
   <211> 26
   <212> DNA
   <213> Artificial
<220>
   <223> An artificially synthesized primer sequence
<400> 21
   aggcgattaa gttgggtaac gccagg 26
<210> 22
   <211> 26
   <212> DNA
   <213> Artificial
<220>
   <223> An artificially synthesized primer sequence
<400> 22
   ccagtcacga cgttgtaaaa cgacgg 26
<210> 23
   <211> 936
   <212> PRT
   <213> Mus musculus
<400> 23
   Met Ala Leu Leu Cys Gly Leu Gly Ser Gly Gly Met Glu Ala Leu Thr
   1               5                   10                  15
   Thr Gln Leu Gly Pro Gly Arg Glu Gly Ser Ser Ser Pro Asn Ser Lys
               20                  25                  30
   Gln Glu Leu Gln Pro Tyr Ser Gly Ser Ser Ala Leu Lys Pro Asn Gln
           35                  40                  45
   Val Gly Glu Thr Ser Leu Tyr Gly Val Pro Ile Val Ser Leu Val Ile
       50                  55                  60
   Asp Gly Gln Glu Arg Leu Cys Leu Ala Gln Ile Ser Asn Thr Leu Leu
   65                  70                  75                  80
   Lys Asn Tyr Ser Tyr Asn Glu Ile His Asn Arg Arg Val Ala Leu Gly
   85 90 95
   Ile Thr Cys Val Gln Cys Thr Pro Val Gln Leu Glu Ile Leu Arg Arg
   100 105 110
   Ala Gly Ala Met Pro Ile Ser Ser Arg Arg Cys Gly Met Ile Thr Lys
   115 120 125
   Arg Glu Ala Glu Arg Leu Cys Lys Ser Phe Leu Gly Glu His Lys Pro
   130 135 140
   Pro Lys Leu Pro Glu Asn Phe Ala Phe Asp Val Val His Glu Cys Ala
   145 150 155 160
   Trp Gly Ser Arg Gly Ser Phe Ile Pro Ala Arg Tyr Asn Ser Ser Arg
   165 170 175
   Ala Lys Cys Ile Lys Cys Gly Tyr Cys Ser Met Tyr Phe Ser Pro Asn
   180 185 190
   Lys Phe Ile Phe His Ser His Arg Thr Pro Asp Ala Lys Tyr Thr Gln
   195 200 205
   Pro Asp Ala Ala Asn Phe Asn Ser Trp Arg Arg His Leu Lys Leu Ser
   210 215 220
   Asp Lys Ser Ala Thr Asp Glu Leu Ser His Ala Trp Glu Asp Val Lys
   225 230 235 240
   Ala Met Phe Asn Gly Gly Thr Arg Lys Arg Thr Phe Ser Leu Gln Gly
   245 250 255
   Gly Gly Gly Gly Gly Ala Asn Ser Gly Ser Gly Gly Ala Gly Lys Gly
   260 265 270
   Gly Ala Gly Gly Gly Gly Gly Pro Gly Cys Gly Ser Glu Met Ala Pro
   275 280 285
   Gly Pro Pro Pro His Lys Ser Leu Arg Cys Gly Glu Asp Glu Ala Ala
   290 295 300
   Gly Pro Pro Gly Pro Pro Pro Pro His Pro Gln Arg Ala Leu Gly Leu
   305 310 315 320
   Ala Ala Ala Ala Ser Gly Pro Ala Gly Pro Gly Gly Pro Gly Gly Ser
   325 330 335
   Ala Gly Val Arg Ser Tyr Pro Val Ile Pro Val Pro Ser Lys Gly Phe
   340 345 350
   Gly Leu Leu Gln Lys Leu Pro Pro Pro Leu Phe Pro His Pro Tyr Gly
   355 360 365
   Phe Pro Thr Ala Phe Gly Leu Cys Pro Lys Lys Asp Asp Pro Val Leu
   370 375 380
   Val Ala Gly Glu Pro Lys Gly Gly Pro Gly Thr Gly Ser Ser Gly Gly
   385 390 395 400
   Ala Gly Thr Ala Ala Gly Ala Gly Gly Pro Gly Ala Gly His Leu Pro
   405 410 415
   Pro Gly Ala Gly Pro Gly Pro Gly Gly Gly Thr Met Phe Trp Gly His
   420 425 430
   Gln Pro Ser Gly Ala Ala Lys Asp Ala Ala Ala Val Ala Ala Ala Ala
   435 440 445
   Ala Ala Ala Thr Val Tyr Pro Thr Phe Pro Met Phe Trp Pro Ala Ala
   450 455 460
   Gly Ser Leu Pro Val Pro Pro Tyr Pro Ala Ala Gln Ser Gln Ala Lys
   465 470 475 480
   Ala Val Ala Ala Ala Val Ala Ala Ala Ala Ala Ala Ala Ala Ala Ala
   485 490 495
   Ala Gly Gly Gly Gly Pro Glu Ser Leu Asp Gly Ala Glu Pro Ala Lys
   500 505 510
   Glu Gly Ser Leu Gly Thr Glu Glu Arg Cys Pro Ser Ala Leu Ser Arg
   515 520 525
   Gly Pro Leu Asp Glu Asp Gly Ala Asp Glu Ala Leu Pro Pro Ser Leu
   530 535 540
   Gly Pro Leu Pro Pro Pro Pro Pro Pro Pro Ala Arg Lys Ser Ser Tyr
   545 550 555 560
   Val Ser Ala Phe Arg Pro Val Val Lys Asp Ala Glu Ser Ile Ala Lys
   565 570 575
   Leu Tyr Gly Ser Ala Arg Glu Ala Tyr Gly Ser Gly Pro Ala Arg Gly
   580 585 590
   Pro Val Pro Gly Thr Gly Thr Gly Gly Gly Tyr Val Ser Pro Asp Phe
   595 600 605
   Leu Ser Glu Gly Ser Ser Ser Tyr His Ser Ala Ser Pro Asp Val Asp
   610 615 620
   Thr Ala Asp Glu Pro Glu Val Asp Val Glu Ser Asn Arg Phe Pro Asp
   625 630 635 640
   Glu Glu Gly Ala Gln Asp Asp Thr Glu Pro Arg Ala Pro Ser Thr Gly
   645 650 655
   Gly Gly Pro Asp Gly Asp Gln Pro Ala Gly Pro Pro Ser Val Thr Ser
   660 665 670
   Ser Gly Ala Asp Gly Pro Thr Asp Ser Ala Asp Gly Asp Ser Pro Arg
   675 680 685
   Pro Arg Arg Arg Leu Gly Pro Pro Pro Ala Ile Arg Ser Ala Phe Gly
   690 695 700
   Asp Leu Val Ala Asp Asp Val Val Arg Arg Thr Glu Arg Ser Pro Pro
   705 710 715 720
   Ser Gly Gly Tyr Glu Leu Arg Glu Pro Cys Gly Pro Leu Gly Gly Pro
   725 730 735
   Gly Ala Ala Lys Val Tyr Ala Pro Glu Arg Asp Glu His Val Lys Ser
   740 745 750
   Thr Ala Val Ala Ala Ala Leu Gly Pro Ala Ala Ser Tyr Leu Cys Thr
   755 760 765
   Pro Glu Thr His Glu Pro Asp Lys Glu Asp Asn His Ser Thr Thr Ala
   770 775 780
   Asp Asp Leu Glu Thr Arg Lys Ser Phe Ser Asp Gln Arg Ser Val Ser
   785 790 795 800
   Gln Pro Ser Pro Ala Asn Thr Asp Arg Gly Glu Asp Gly Leu Thr Leu
   805 810 815
   Asp Val Thr Gly Thr Gln Leu Val Glu Lys Asp Ile Glu Asn Leu Ala
   820 825 830
   Arg Glu Glu Leu Gln Lys Leu Leu Leu Glu Gln Met Glu Leu Arg Lys
   835 840 845
   Lys Leu Glu Arg Glu Phe Gln Ser Leu Lys Asp Asn Phe Gln Asp Gln
   850 855 860
   Met Lys Arg Glu Leu Ala Tyr Arg Glu Glu Met Val Gln Gln Leu Gln
   865 870 875 880
   Ile Val Arg Asp Thr Leu Cys Asn Glu Leu Asp Gln Glu Arg Lys Ala
   885 890 895
   Arg Tyr Ala Ile Gln Gln Lys Leu Lys Glu Ala His Asp Ala Leu His
   900 905 910
   His Phe Ser Cys Lys Met Leu Thr Pro Arg His Cys Thr Gly Asn Cys
   915 920 925
   Ser Phe Lys Pro Pro Leu Leu Pro
   930 935
<210> 24
   <211> 725
   <212> PRT
   <213> Mus musculus
<400> 24
   Met Glu Ala Ala Ala Ala Gly Arg Gly Gly Phe Gln Gln Pro Gly Leu
   1 5 10 15
   Gln Lys Thr Leu Glu Gln Phe His Leu Ser Ser Met Ser Ser Leu Gly
   20 25 30
   Gly Pro Ala Val Ser Arg Arg Ala Gly Gln Glu Ala Tyr Lys Lys Glu
   35 40 45
   Ser Ala Lys Glu Ala Gly Ala Ala Thr Val Pro Ala Pro Val Pro Thr
   50 55 60
   Ala Ala Glu Pro Pro Pro Val Leu His Leu Pro Ala Ile Gln Pro Pro
   65 70 75 80
   Pro Pro Val Leu Pro Gly Pro Phe Phe Met Pro Ser Asp Arg Ser Thr
   85 90 95
   Glu Arg Cys Glu Thr Val Leu Glu Gly Glu Thr Ile Ser Cys Phe Val
   100 105 110
   Val Gly Gly Glu Lys Arg Leu Cys Leu Pro Gln Ile Leu Asn Ser Val
   115 120 125
   Leu Arg Asp Phe Ser Leu Gln Gln Ile Asn Ser Val Cys Asp Glu Leu
   130 135 140
   His Ile Tyr Cys Ser Arg Cys Thr Ala Asp Gln Leu Glu Ile Leu Lys
   145 150 155 160
   Val Met Gly Ile Leu Pro Phe Ser Ala Pro Ser Cys Gly Leu Ile Thr
   165 170 175
   Lys Thr Asp Ala Glu Arg Leu Cys Asn Ala Leu Leu Tyr Gly Gly Ala
   180 185 190
   Tyr Pro Pro Pro Cys Lys Lys Glu Leu Ala Ala Ser Leu Ala Leu Gly
   195 200 205
   Leu Glu Leu Ser Glu Arg Ser Val Arg Val Tyr His Glu Cys Phe Gly
   210 215 220
   Lys Cys Lys Gly Leu Leu Val Pro Glu Leu Tyr Ser Ser Pro Ser Ala
   225 230 235 240
   Ala Cys Ile Gln Cys Leu Asp Cys Arg Leu Met Tyr Pro Pro His Lys
   245 250 255
   Phe Val Val His Ser His Lys Ala Leu Glu Asn Arg Thr Cys His Trp
   260 265 270
   Gly Phe Asp Ser Ala Asn Trp Arg Ala Tyr Ile Leu Leu Ser Gln Asp
   275 280 285
   Tyr Thr Gly Lys Glu Glu Gln Ala Arg Leu Gly Arg Cys Leu Asp Asp
   290 295 300
   Val Lys Glu Lys Phe Asp Tyr Ala Asn Lys Tyr Lys Arg Arg Val Pro
   305 310 315 320
   Arg Val Ser Glu Pro Pro Ala Ser Ile Arg Pro Lys Thr Asp Asp Thr
   325 330 335
   Ser Ser Gln Ser Pro Ala Ser Ser Glu Lys Asp Lys Gln Ser Thr Trp
   340 345 350
   Leu Arg Thr Leu Ala Gly Ser Ser Asn Lys Ser Leu Gly Cys Thr His
   355 360 365
   Pro Arg Gln Arg Leu Ser Ala Phe Arg Pro Trp Ser Pro Ala Val Ser
   370 375 380
   Ala Ser Glu Lys Glu Thr Ser Pro His Leu Pro Ala Leu Ile Arg Asp
   385 390 395 400
   Ser Phe Tyr Ser Tyr Lys Ser Phe Glu Thr Ala Val Ala Pro Asn Val
   405 410 415
   Ala Leu Ala Pro Pro Thr Gln Gln Lys Val Val Asn Ser Pro Pro Cys
   420 425 430
   Thr Thr Val Val Ser Arg Ala Pro Glu Pro Leu Thr Thr Cys Ile Gln
   435 440 445
   Pro Arg Lys Arg Lys Leu Thr Leu Asp Thr Ala Gly Ala Pro Asp Met
   450 455 460
   Leu Thr Pro Val Ala Ala Ala Glu Glu Asp Lys Asp Ser Glu Ala Glu
   465 470 475 480
   Val Glu Val Glu Ser Arg Glu Glu Phe Thr Ser Ser Leu Ser Ser Leu
   485 490 495
   Ser Ser Pro Ser Phe Thr Ser Ser Ser Ser Ala Lys Asp Leu Ser Ser
   500 505 510
   Pro Gly Met His Ala Pro Pro Val Val Ala Pro Asp Ala Ala Ala His
   515 520 525
   Val Asp Ala Pro Ser Gly Leu Glu Ala Glu Leu Glu His Leu Arg Gln
   530 535 540
   Ala Leu Glu Gly Gly Leu Asp Thr Lys Glu Ala Lys Glu Lys Phe Leu
   545 550 555 560
   His Glu Val Val Lys Met Arg Val Lys Gln Glu Glu Lys Leu Thr Ala
   565 570 575
   Ala Leu Gln Ala Lys Arg Thr Leu His Gln Glu Leu Glu Phe Leu Arg
   580 585 590
   Val Ala Lys Lys Glu Lys Leu Arg Glu Ala Thr Glu Ala Lys Arg Asn
   595 600 605
   Leu Arg Lys Glu Ile Glu Arg Leu Arg Ala Glu Asn Glu Lys Lys Met
   610 615 620
   Lys Glu Ala Asn Glu Ser Arg Val Arg Leu Lys Arg Glu Leu Glu Gln
   625 630 635 640
   Ala Arg Gln Val Arg Val Cys Asp Lys Gly Cys Glu Ala Gly Arg Leu
   645 650 655
   Arg Ala Lys Tyr Ser Ala Gln Val Glu Asp Leu Gln Ala Lys Leu Gln
   660 665 670
   His Ala Glu Ala Asp Arg Glu Gln Leu Arg Ala Asp Leu Leu Arg Glu
   675 680 685
   Arg Glu Ala Arg Glu His Leu Glu Lys Val Val Arg Glu Leu Gln Glu
   690 695 700
   Gln Leu Arg Pro Arg Pro Arg Pro Glu His Pro Gly Gly Glu Ser Asn
   705 710 715 720
   Ala Glu Leu Gly Pro
   725
<210> 25
   <211> 674
   <212> PRT
   <213> Mus musculus
<400> 25
   Met Glu Asn Leu Gln Ser Lys Phe Ser Leu Val Gln Gly Ser Asn Lys
   1 5 10 15
   Lys Leu Asn Gly Met Glu Asp Asp Gly Ser Pro Pro Val Lys Lys Met
   20 25 30
   Met Thr Asp Ile His Ala Asn Gly Lys Thr Leu Thr Lys Val Lys Lys
   35 40 45
   Glu His Leu Asp Asp Tyr Gly Asp Ala Ser Val Glu Pro Asp Arg Ala
   50 55 60
   Arg Lys Arg Asn Arg Val Ser Leu Pro Glu Thr Leu Asn Leu Asn Pro
   65 70 75 80
   Ser Leu Lys His Thr Leu Ala Gln Phe His Leu Ser Ser Gln Ser Ser
   85 90 95
   Leu Gly Gly Pro Ala Ala Phe Ser Ala Arg Tyr Ser Gln Glu Ser Met
   100 105 110
   Ser Pro Thr Val Phe Leu Pro Leu Pro Ser Pro Gln Val Leu Pro Gly
   115 120 125
   Thr Leu Leu Ile Pro Ser Asp Ser Ser Thr Glu Leu Thr Gln Thr Leu
   130 135 140
   Leu Glu Gly Glu Thr Ile Ser Cys Phe Gln Val Gly Gly Glu Lys Arg
   145 150 155 160
   Leu Cys Leu Pro Gln Val Leu Asn Ser Val Leu Arg Glu Phe Ser Leu
   165 170 175
   Gln Gln Ile Asn Thr Val Cys Asp Glu Leu Tyr Ile Tyr Cys Ser Arg
   180 185 190
   Cys Thr Ser Asp Gln Leu His Ile Leu Lys Val Ser Gly Ile Leu Pro
   195 200 205
   Phe Asn Ala Pro Ser Cys Gly Leu Ile Thr Leu Thr Asp Ala Gln Arg
   210 215 220
   Leu Cys Asn Thr Leu Leu Arg Pro Arg Thr Phe Pro Gln Asn Gly Ser
   225 230 235 240
   Ile Leu Pro Ala Lys Ser Ser Leu Ala Gln Leu Lys Glu Thr Gly Ser
   245 250 255
   Ala Phe Glu Val Glu His Glu Cys Leu Gly Lys Cys Gln Gly Leu Phe
   260 265 270
   Ala Pro Gln Phe Tyr Val Gln Pro Asp Ala Pro Cys Ile Gln Cys Leu
   275 280 285
   Glu Cys Cys Gly Met Phe Ala Pro Gln Thr Phe Val Met His Ser His
   290 295 300
   Arg Ser Pro Asp Lys Arg Thr Cys His Trp Gly Phe Glu Ser Ala Lys
   305 310 315 320
   Trp His Cys Tyr Leu His Val Asn Gln Lys Tyr Leu Gly Thr Pro Glu
   325 330 335
   Glu Lys Lys Leu Lys Ile Ile Leu Glu Glu Met Lys Glu Lys Phe Ser
   340 345 350
   Met Arg Asn Gly Lys Arg Ile Gln Ser Lys Thr Asp Thr Pro Ser Gly
   355 360 365
   Met Glu Leu Pro Ser Trp Tyr Pro Val Ile Lys Gln Glu Gly Asp His
   370 375 380
   Val Pro Gln Thr His Ser Phe Leu His Pro Ser Tyr Tyr Leu Tyr Met
   385 390 395 400
   Cys Asp Lys Val Val Ala Pro Asn Val Ser Leu Thr Ser Ala Ala Ser
   405 410 415
   Gln Ser Lys Glu Ala Thr Lys Ala Glu Thr Ser Lys Ser Thr Ser Lys
   420 425 430
   Gln Ser Glu Lys Pro His Glu Ser Ser Gln His Gln Lys Thr Val Ser
   435 440 445
   Tyr Pro Asp Val Ser Leu Glu Glu Gln Glu Lys Met Asp Leu Lys Thr
   450 455 460
   Ser Arg Glu Leu Tyr Ser Cys Leu Asp Ser Ser Ile Ser Asn Asn Ser
   465 470 475 480
   Thr Ser Arg Lys Lys Ser Glu Ser Ala Val Cys Ser Leu Val Arg Gly
   485 490 495
   Thr Ser Lys Arg Asp Ser Glu Asp Ser Ser Pro Leu Leu Val Arg Asp
   500 505 510
   Gly Glu Asp Asp Lys Gly Lys Ile Met Glu Asp Val Met Arg Thr Tyr
   515 520 525
   Val Arg Gln Gln Glu Lys Leu Asn Ser Ile Leu Gln Arg Lys Gln Gln
   530 535 540
   Leu Gln Met Glu Val Glu Met Leu Ser Ser Ser Lys Ala Met Lys Glu
   545 550 555 560
   Leu Thr Glu Glu Gln Gln Asn Leu Gln Lys Glu Leu Glu Ser Leu Gln
   565 570 575
   Ser Glu His Ala Gln Arg Met Glu Glu Phe Tyr Ile Glu Gln Arg Asp
   580 585 590
   Leu Glu Lys Lys Leu Glu Gln Val Met Gln Gln Lys Cys Thr Cys Asp
   595 600 605
   Ser Thr Leu Glu Lys Asp Arg Glu Ala Glu Tyr Ala Gly Gln Leu Ala
   610 615 620
   Glu Leu Arg Gln Arg Leu Asp His Ala Glu Ala Asp Gln Arg Glu Leu
   625 630 635 640
   Gln Asp Glu Leu Arg Gln Glu Arg Glu Ala Arg Gln Lys Leu Glu Met
   645 650 655
   Met Ile Lys Glu Leu Lys Leu Gln Ile Gly Lys Ser Ser Lys Pro Ser
   660 665 670
   Lys Asp
<210> 26
   <211> 751
   <212> PRT
   <213> Mus musculus
<400> 26
   Met Ala Val Pro Ala Ala Leu Ile Pro Pro Thr Gln Leu Val Pro Pro
   1               5                   10                  15
   Gln Pro Pro Ile Ser Thr Ser Ala Ser Ser Ser Gly Thr Thr Thr Ser
               20                  25                  30
   Thr Ser Ser Ala Thr Ser Ser Pro Ala Pro Ser Ile Gly Pro Pro Ala
           35                  40                  45
   Ser Ser Gly Pro Thr Leu Phe Arg Pro Glu Pro Ile Ala Ser Ser Ala
       50                  55                  60
   Ser Ser Ser Ala Ala Ala Thr Val Thr Ser Pro Gly Gly Gly Gly Gly
   65                  70                  75                  80
   Gly Ser Gly Gly Gly Gly Gly Ser Gly Gly Asn Gly Gly Gly Gly Gly
                   85                  90                  95
   Ser Asn Cys Asn Pro Ser Leu Ala Ala Gly Ser Ser Gly Gly Gly Val
               100                 105                 110
   Ser Ala Gly Gly Gly Gly Ala Ser Ser Thr Pro Ile Thr Ala Ser Thr
           115                 120                 125
   Gly Ser Ser Ser Ser Ser Ser Ser Ser Ser Ser Ser Ser Ser Ser Ser
       130                 135                 140
   Ser Ser Ser Ser Ser Ser Ser Ser Ser Ser Ser Ser Cys Gly Pro Leu
   145                 150                 155                 160
   Pro Gly Lys Pro Val Tyr Ser Thr Pro Ser Pro Val Glu Asn Thr Pro
   165 170 175
   Gln Asn Asn Glu Cys Lys Met Val Asp Leu Arg Gly Ala Lys Val Ala
   180 185 190
   Ser Phe Thr Val Glu Gly Cys Glu Leu Ile Cys Leu Pro Gln Ala Phe
   195 200 205
   Asp Leu Phe Leu Lys His Leu Val Gly Gly Leu His Thr Val Tyr Thr
   210 215 220
   Lys Leu Lys Arg Leu Glu Ile Thr Pro Val Val Cys Asn Val Glu Gln
   225 230 235 240
   Val Arg Ile Leu Arg Gly Leu Gly Ala Ile Gln Pro Gly Val Asn Arg
   245 250 255
   Cys Lys Leu Ile Ser Arg Lys Asp Phe Glu Thr Leu Tyr Asn Asp Cys
   260 265 270
   Thr Asn Ala Ser Ser Arg Pro Gly Arg Pro Pro Lys Arg Thr Gln Ser
   275 280 285
   Val Thr Ser Pro Glu Asn Ser His Ile Met Pro His Ser Val Pro Gly
   290 295 300
   Leu Met Ser Pro Gly Ile Ile Pro Pro Thr Gly Leu Thr Ala Ala Ala
   305 310 315 320
   Ala Ala Ala Ala Ala Ala Thr Asn Ala Ala Ile Ala Glu Ala Met Lys
   325 330 335
   Val Lys Lys Ile Lys Leu Glu Ala Met Ser Asn Tyr His Ala Ser Asn
   340 345 350
   Asn Gln His Gly Ala Asp Ser Glu Asn Gly Asp Met Asn Ser Ser Val
   355 360 365
   Gly Ser Ser Gly Gly Ser Trp Asp Lys Glu Thr Leu His Ser Pro Pro
   370 375 380
   Ser Gln Gly Ser Gln Ala Pro Val Ala His Ala Arg Met Pro Ala Ala
   385 390 395 400
   Phe Ser Leu Pro Val Ser His Pro Leu Asn His Leu Gln His Ser His
   405 410 415
   Leu Pro Pro Asn Gly Leu Glu Leu Pro Phe Met Met Met Pro His Pro
   420 425 430
   Leu Ile Pro Val Ser Leu Pro Pro Ala Ser Val Thr Met Ala Met Ser
   435 440 445
   Gln Met Asn His Leu Ser Thr Ile Ala Asn Met Ala Ala Ala Ala Gln
   450 455 460
   Val Gln Ser Pro Pro Ser Arg Val Glu Thr Ser Val Ile Lys Glu Arg
   465 470 475 480
   Val Pro Asp Ser Pro Ser Pro Ala Pro Ser Leu Glu Glu Gly Arg Arg
   485 490 495
   Pro Gly Ser His Pro Ser Ser His Arg Ser Ser Ser Val Ser Ser Ser
   500 505 510
   Pro Ala Arg Thr Glu Ser Ser Ser Asp Arg Ile Pro Val His Gln Asn
   515 520 525
   Gly Leu Ser Met Asn Gln Met Leu Met Gly Leu Ser Pro Asn Val Leu
   530 535 540
   Pro Gly Pro Lys Glu Gly Asp Leu Ala Gly His Asp Met Gly His Glu
   545 550 555 560
   Ser Lys Arg Ile His Ile Glu Lys Asp Glu Thr Pro Leu Ser Thr Pro
   565 570 575
   Thr Ala Arg Asp Ser Ile Asp Lys Leu Ser Leu Thr Gly His Gly Gln
   580 585 590
   Pro Leu Pro Pro Gly Phe Pro Ser Pro Phe Leu Phe Pro Asp Gly Leu
   595 600 605
   Ser Ser Ile Glu Thr Leu Leu Thr Asn Ile Gln Gly Leu Leu Lys Val
   610 615 620
   Ala Ile Asp Asn Ala Arg Ala Gln Glu Lys Gln Val Gln Leu Glu Lys
   625 630 635 640
   Thr Glu Leu Lys Met Asp Phe Leu Arg Glu Arg Glu Leu Arg Glu Thr
   645 650 655
   Leu Glu Lys Gln Leu Ala Met Glu Gln Lys Asn Arg Ala Ile Val Gln
   660 665 670
   Lys Arg Leu Lys Lys Glu Lys Lys Ala Lys Arg Lys Leu Gln Glu Ala
   675 680 685
   Leu Glu Phe Glu Thr Lys Arg Arg Glu Gln Ala Glu Gln Thr Leu Lys
   690 695 700
   Gln Ala Ala Ser Ala Asp Ser Leu Arg Val Leu Asn Asp Ser Leu Thr
   705 710 715 720
   Pro Glu Ile Glu Ala Asp Arg Ser Gly Gly Arg Ala Asp Ala Glu Arg
   725 730 735
   Thr Ile Gln Asp Gly Arg Leu Tyr Leu Lys Thr Thr Val Met Tyr
   740 745 750
<210> 27
   <211> 26
   <212> DNA
   <213> Artificial
<220>
   <223> An artificially synthesized primer sequence
<400> 27
   ggacatggct tcttcatcac agattc 26
<210> 28
   <211> 26
   <212> DNA
   <213> Artificial
<220>
   <223> An artificially synthesized primer sequence
<400> 28
   gtaactgttg caccatctct tctcgg 26
<210> 29
   <211> 26
   <212> DNA
   <213> Artificial
<220>
   <223> An artificially synthesized primer sequence
<400> 29
   atgcagagag catcgctaag ctctac 26
<210> 30
   <211> 26
   <212> DNA
   <213> Artificial
<220>
   <223> An artificially synthesized primer sequence
<400> 30
   aagcggttgg actctacgtc cacctc 26
<210> 31
   <211> 26
   <212> DNA
   <213> Artificial
<220>
   <223> An artificially synthesized primer sequence
<400> 31
   acgacccctt cattgacctc aactac 26
<210> 32
   <211> 26
   <212> DNA
   <213> Artificial
<220>
   <223> An artificially synthesized primer sequence
<400> 32
   ccagtagact ccacgacata ctcagc 26

## Description

### Technical Field

The present invention relates to Corl2 expressed specifically in Purkinje cells, and uses thereof.

### Background Art

The brain functions as a complex network formed by a great variety of neurons. Its failure may result in various neurological diseases. Transplantation and *in vivo* regeneration therapy are currently investigated as therapeutic methods. The most important thing in these therapeutic methods is to first correctly identify various neurons. Furthermore, controlling the regeneration is difficult without understanding the differentiation mechanism of individual neurons.

The cerebellum performs for smooth motor function, such as regulation of balance, posture, and voluntary movement. The failure of cerebellar function due to cerebellar tumor, cerebellar vermis degeneration caused by chronic alcoholism, or such results in dynamic ataxia and balance disorder. Functional recovery after such damages can be achieved by replenishing lost neurons and reconstituting the network.

There are about five types of neurons in the cerebellum including Purkinje cell. Neurotransmission is achieved when proper network is formed by the respective neurons according to organogenic program. Thus, it is important to identify cell populations in transplantation material in detail for the safety and therapeutic effect in regenerative therapy. Furthermore, to improve therapeutic effect, it is also considered important to induce *in vitro* or *in vivo* differentiation of only the neurons that are needed, and for this purpose, it would be essential to identify individual neurons in detail.

Purkinje cells in the mature cerebellum can be easily identified based on their localization and morphology. Calbindin and RORalpha are known to serve as molecular markers for Purkinje cells. Purkinje cells can be properly identified using these markers in combination. However, calbindin is a marker expressed in mature Purkinje cells, and is therefore not suitable for identifying Purkinje cells in the premature cerebellum or *in vitro* differentiation-induced cell populations. Meanwhile, RORalpha is assumed to be expressed in relatively premature Purkinje cells after development; however, RORalpha has poor specificity because it is expressed not only in Purkinje cells but also in other cells even within the cerebellum. Therefore, if a marker specific for Purkinje cell immediately after differentiation can be identified, it is expected to be useful for testing the purity of transplantation material, developing methods for inducing *in vitro* differentiation of Purkinje cells, and so on.

### Disclosure of the Invention

### Problems to Be Solved by the Invention

The present invention was achieved in view of such situation. An objective of the present invention is to discover novel Purkinje cell-specific markers.

### Means to Solve the Problems

To solve these problems, the present inventors tried to isolate novel genes expressed specifically in fetal brain regions. First, the inventors prepared total RNAs from the ventral and dorsal regions of an embryonic day 12.5 mouse mesencephalon, and obtained a cDNA fragment by the subtraction method (N-RDA). Homology search of the cDNA fragment revealed homology to a gene encoding a functionally unknown protein (Genbank accession No.: XM_355050). However, since the sequence deposited in the databank is a sequence deduced from a genomic sequence, the present inventors tried to clone a full-length cDNA sequence for this gene. Analysis of the nucleotide sequence of the amplified fragment obtained by performing RACE with a cDNA library prepared from an embryonic day 12.5 mouse brain revealed that the gene encodes 1,008 amino acids, and it was named Corl2. New homology search was carried out using this Corl2. The result showed that about 850 residues of Corl2 exhibited homology to the deposited XM_355050 sequence described above; however, the remaining ∼150 residues showed no homology at all. Thus, Corl2 identified by the present inventors was demonstrated to be a novel gene.

The present inventors then analyzed the expressions of the novel gene Corl2 in various mouse organs by RT-PCR. The result showed that the Corl2 expression was brain specific and the expression level was higher at fetal stages than in adult. To further analyze the Corl2 expression in the cerebellum, the inventors prepared an anti-Corl2 polyclonal antibody and performed immunostaining. The result obtained using day 12.5 embryos suggested that Corl2 was expressed in the precursor cells of Purkinje cells and the result using postnatal day 12 cerebellum suggested that Corl2 was also specifically expressed in the Purkinje cell layer. This demonstrates that Corl2 is useful as a Purkinje cell marker across all differentiation stages. The present invention thus relates to Corl2 and uses thereof. Specifically, the present invention provides the following inventions:
[1] An isolated polynucleotide of any one of:
   (a) a polynucleotide comprising the nucleotide sequence of SEQ ID NO: 2 or 4;
   (b) a polynucleotide encoding a polypeptide comprising the amino acid sequence of SEQ ID NO:1 or 3; and
   (c) a polynucleotide encoding a polypeptide comprising an amino acid sequence with an addition, deletion, insertion, and/or substitution of one or more amino acids in the amino acid sequence of SEQ ID NO:1 or 3, wherein the number of amino acids added, deleted, inserted, and/or substituted is 10% or less of the total amino acids.
[2] An isolated polynucleotide of any one of:
   (a) a polynucleotide encoding a polypeptide comprising the amino acid sequence of any one of positions 643 to 772, 867 to 886, and 989 to 1008 in the amino acid sequence of SEQ ID NO: 1;
   (b) a polynucleotide encoding a polypeptide comprising the polypeptide encoded by the sequence of positions 2285 to 2669 or 2956 to 3013 in the nucleotide sequence of SEQ ID NO: 2;
   (c) a polynucleotide encoding a polypeptide comprising an amino acid sequence with an addition, deletion, insertion, and/or substitution of one or more amino acids in a polypeptide comprising the amino acid sequence of any one of positions 643 to 772, 867 to 886, and 989 to 1008 in the amino acid sequence of SEQ ID NO: 1, wherein the number of amino acids added, deleted, inserted, and/or substituted is 10% or less of the total amino acids; and
   (d) a polynucleotide encoding a polypeptide comprising a polypeptide encoded by the nucleotide sequence of a polynucleotide that hybridizes under stringent conditions to a polynucleotide comprising the nucleotide sequence of positions 2285 to 2669 or 2956 to 3013 in the nucleotide sequence of SEQ ID NO: 2.
[3] A vector comprising the polynucleotide of [1] or [2].
[4] A transformant retaining the polynucleotide of [1] or [2] or the vector of [3].
[5] An isolated polypeptide of any one of:
   (a) a polypeptide comprising the amino acid sequence of SEQ ID NO: 1 or 3;
   (b) a polypeptide comprising a polypeptide encoded by the nucleotide sequence of SEQ ID NO: 2 or 4; and
   (c) a polypeptide comprising an amino acid sequence with an addition, deletion, insertion, and/or substitution of one or more amino acids in the amino acid sequence of SEQ ID NO: 1 or 3 wherein the number of amino acids added, deleted, inserted, and/or substituted is 10% or less of the total amino acids.
[6] An isolated polypeptide of any one of:
   (a) a polypeptide comprising the amino acid sequence of any one of positions 643 to 772, 867 to 886, and 989 to 1008 in the amino acid sequence of SEQ ID NO: 1;
   (b) a polypeptide comprising a polypeptide encoded by the nucleotide sequence of positions 2285 to 2669 or 2956 to 3013 in the nucleotide sequence of SEQ ID NO: 2;
   (c) a polypeptide comprising an amino acid sequence with an addition, deletion, insertion, and/or substitution of one or more amino acids in a polypeptide comprising the amino acid sequence of any one of positions 643 to 772, 867 to 886, and 989 to 1008 of SEQ ID NO: 1 wherein the number of amino acids added, deleted, inserted, and/or substituted is 10% or less of the total amino acids; and
   (d) a polypeptide comprising a polypeptide encoded by the nucleotide sequence of a polynucleotide that hybridizes under stringent conditions to a polynucleotide comprising the nucleotide sequence of positions 2285 to 2669 or 2956 to 3013 in the nucleotide sequence of SEQ ID NO: 2.
[7] A polynucleotide comprising at least 15 consecutive nucleotides of a nucleotide sequence from positions 357 to 458, 924 to 1478, 1689 to 3128, or 3321 to 3380 of the nucleotide sequence of SEQ ID NO: 2 or 4, or the complementary strand thereof.
[8] The polynucleotide of [7], wherein the polynucleotide that has at least 15 consecutive nucleotides is the polynucleotide of any one of SEQ ID NOs: 15, 17, 18, 27, and 28.
[9] An antibody capable of binding to the polypeptide of [5] or [6].
[10] The antibody of [9], which is capable of binding to a polypeptide comprising any one of:
   (a) a polypeptide comprising the amino acid sequence of any one of positions 1 to 43, 190 to 374, 445 to 924, and 989 to 1008 in the amino acid sequence of SEQ ID NO: 1; and
   (b) a polypeptide comprising an amino acid sequence with an addition, deletion, insertion, and/or substitution of one or more amino acids in a polypeptide comprising the amino acid sequence of any one of positions 1 to 43, 190 to 374, 445 to 924, and 989 to 1008 in the amino acid sequence of SEQ ID NO: 1.
[11] The antibody of [9], which is capable of binding to a polypeptide that comprises a polypeptide comprising the sequence of positions 836 to 924 of the amino acid sequence of SEQ ID NO: 1.
[12] A method for producing the polypeptide of [5] or [6], which comprises the step of culturing the transformant of [4] and collecting a protein from the transformant or culture supernatant.
[13] A method for identifying Purkinje cell, which comprises the step of contacting a test sample with the polynucleotide of [7] or [8].
[14] A method for identifying Purkinje cell, which comprises the step of contacting a test sample with the antibody of any one of [9] to [11].
[15] A method for separating Purkinje cell, which comprises the step of contacting a test sample with the antibody of any one of [9] to [11].
[16] An agent for identifying Purkinje cell, which comprises the polynucleotide of [7] or [8], or the antibody of any one of [9] to [11].
[17] An agent for separating Purkinje cell, which comprises the antibody of any one of [9] to [11].
[18] A kit for identifying Purkinje cell, which comprises the polynucleotide of [7] or [8], or the antibody of any one of [9] to [11].
[19] A kit for separating Purkinje cell, which comprises the antibody of any one of [9] to [11].

### Brief Description of the Drawings

Fig. 1 shows the structure of the Corl2 protein and its relation with related genes. The percentages indicated in Fig. 1A correspond to homologies of the respective domains.
Fig. 2 shows the result of detecting Corl2 mRNA expression in various adult tissues.
Fig. 3 assesses Corl2 expression in the brain regions of an embryonic day 12.5 mouse by immunostaining.
Fig. 4 assesses Corl2 expression in cerebellar regions of a postnatal day 12 mouse by immunostaining. Arrowheads in the photographs indicate Purkinje cells.

### Best Mode for Carrying Out the Invention

The present invention relates to polynucleotides encoding the novel protein Corl2. Corl2 is a protein specifically expressed in Purkinje cells. The present inventors for the first time isolated Corl2 cDNA. The amino acid sequence of mouse Corl2 is shown in SEQ ID NO: 1, and the nucleotide sequence of Corl2 is shown in SEQ ID NO: 2. In SEQ ID NO: 2, the start codon is the ATG at positions 357 to 359, and the stop codon is the TAA at positions 3381 to 3383. The putative amino acid sequence of human Corl2 is also shown in SEQ ID NO: 3, and the putative nucleotide sequence of human Corl2 is shown in SEQ ID NO: 4. In SEQ ID NO: 4, the start codon is the ATG at positions 1 to 3, and the stop codon is the TAA at positions 3043 to 3045.

Herein, the "polynucleotide" refers to two or more nucleotides linked together, including general "oligonucleotides" and both DNAs and RNAs. Herein, the "polypeptide" refers to two or more amino acids linked together, including general "peptides", "oligopeptides", and "proteins". Herein, "isolation" means placement in a state different from the natural state by artificial treatment.

Polynucleotides encoding Corl2 can be prepared by methods known to those skilled in the art, for example, by RT-PCR using mRNA prepared from mouse or human cerebellar tissues, or mouse or human embryos, and primers synthesized based on the nucleotide sequence of SEQ ID NO: 2 or 4. Alternatively, the polynucleotides can be prepared, for example, from a mouse or human cerebellar cDNA library by hybridization using as a probe the nucleotide sequence of SEQ ID NO: 2 or 4, or a portion thereof. Alternatively, the polynucleotides can be obtained, for example, by synthesizing the nucleotide sequence of SEQ ID NO: 2 or 4 using commercially available nucleic acid synthesizers.

The polynucleotides encoding polypeptides similar to Corl2 are also included in the present invention. Such polynucleotides encoding polypeptides similar to Corl2 include polynucleotides that hybridize under stringent conditions to polynucleotides having the nucleotide sequence of SEQ ID NO: 2 or 4. Furthermore, polynucleotides encoding amino acid sequences with an addition, deletion, insertion, and/or substitution of one or more amino acids in the amino acid sequence of SEQ ID NO: 1 or 3 are also included in the polynucleotides encoding polypeptides similar to Corl2. These are spontaneous mutants of Corl2 in mammals such as rat, mouse, guinea pig, goat, donkey, horse, sheep, rabbit, dog, chimpanzee, and human, preferably mouse or human; Corl2 homologs and orthologs from various animals (for example, birds, and mammals such as rats, guinea pigs, goats, donkeys, horses, sheep, rabbits, dogs, and chimpanzees), and mutants thereof; Corl2 comprising SNPs and the above-described homologs and orthologs comprising SNPs, and mutants thereof; and artificially generated mutants.

Herein, "amino acid substitution" refers to a mutation in which one or more amino acid residues in a sequence are changed to a different type of amino acid residue. When the amino acid sequence encoded by a polynucleotide of the present invention is altered by such a substitution, a conservative substitution is preferably carried out if the function of the protein is to be maintained. Conservative substitution means altering a sequence so that it encodes an amino acid that has properties similar to those of the amino acid before substitution. Amino acids can be classified, based on their properties, into non-polar amino acids (Ala, Ile, Leu, Met, Phe, Pro, Trp, Val), non-charged amino acids (Asn, Cys, Gln, Gly, Ser, Thr, Tyr), acidic amino acids (Asp, Glu), basic amino acids (Arg, His, Lys), neutral amino acids (Ala, Asn, Cys, Gln, Gly, Ile, Leu, Met, Phe, Pro, Ser, Thr, Trp, Tyr, Val), aliphatic amino acids (Ala, Gly), branched amino acids (Ile, Leu, Val), hydroxyamino acids (Ser, Thr), amide-type amino acids (Gln, Asn), sulfur-containing amino acids (Cys, Met), aromatic amino acids (His, Phe, Trp, Tyr), heterocyclic amino acids (His, Trp), imino acids (Pro, 4Hyp), and such. In particular, substitutions among Ala, Val, Leu, and Ile; Ser and Thr; Asp and Glu; Asn and Gln; Lys and Arg; and Phe and Tyr, are preferable in order to maintain protein properties. There are no particular limitations on the number and sites of the mutated amino acids, as long as the amino acid encoded by the polynucleotide has Corl2 antigenicity.

A polynucleotide encoding an amino acid sequence, in which one or more amino acids are deleted, inserted, substituted, and/or added to the sequence of SEQ ID NO: lor 3, can be prepared according to methods such as site-directed mutagenesis described in "Molecular Cloning, A Laboratory Manual 2nd ed." (Cold Spring Harbor Press (1989)); "Current Protocols in Molecular Biology" (John Wiley & Sons (1987-1997), especially Sections 8.1-8.5); Hashimoto-Goto et al., Gene (1995) 152, 271-5; Kunkel, Proc. Natl. Acad. Sci. USA (1985) 82, 488-92; Kramer and Fritz, Method. Enzymol. (1987) 154, 350-67; Kunkel, Method. Enzymol. (1988) 85, 2763-6; and others.

In the present invention, the number of amino acids added, deleted, inserted, and/or substituted is not particularly limited, as long as the polypeptide has the same function as Corl2 or cross-reacts with Corl2. In general, the number is 10% or less of the total amino acids, preferably 3% or less of the total amino acids, more preferably 1% or less of the total amino acids, even more preferably 9 or less, still more preferably 6 or less, yet the most preferably 1 or 2.

In the present invention, the polynucleotides encoding polypeptides similar to Corl2 can be prepared by means known to those skilled in the art. The polynucleotides can be prepared, for example, from a mouse or human cerebellar cDNA library by hybridization under stringent conditions using as a probe the nucleotide sequence of SEQ ID NO: 2 or 4, or a portion thereof.

Those skilled in the art can appropriately select the above stringent hybridization conditions. For example, pre-hybridization is carried out in a hybridization solution containing 25% formamide, or 50% formamide under more stringent conditions, and 4x SSC, 50 mM Hepes (pH7.0), 10x Denhardt's solution, and 20 µg/ml denatured salmon sperm DNA at 42°C overnight. A labeled probe is then added to the solution and hybridization is carried out by incubation at 42°C overnight. Post-hybridization washes are carried out at different levels of stringency, including the moderately stringent "1x SSC, 0.1% SDS, 37°C", highly stringent "0.5x SSC, 0.1% SDS, 42°C", and more highly stringent "0.2x SSC, 0.1% SDS, 65°C" conditions. As the stringency of the post-hybridization washes increases, polynucleotides with greater homology to the probe sequence are expected to be isolated. The above-described combinations of SSC, SDS, and temperature are merely examples of washing conditions. Those skilled in the art can achieve the same stringencies as those described above by appropriately combining the above factors or others (such as probe concentration, probe length, or hybridization period) that affect hybridization stringency.

Polypeptides encoded by polynucleotides isolated using such hybridization techniques will usually comprise amino acid sequences highly homologous to the polypeptides identified by the present inventors. "High homology" refers to sequence homology of at least 40% or more, preferably 60% or more, further preferably 80% or more, further preferably 90% or more, further preferably at least 95% or more, and further preferably at least 97% or more (for example, 98% to 99%). Amino acid sequence identity can be determined, for example, using the BLAST algorithm of Karlin and Altschul (Proc. Natl. Acad. Sci. USA (1990) 87, 2264-2268; Proc. Natl. Acad. Sci. USA (1993) 90, 5873-5877). A program called BLASTX has been developed based on this algorithm (Altschul et al., J. Mol. Biol. (1990) 215, 403-410). When using BLASTX to analyze amino acid sequence identity, the parameters are, for example, a score of 50 and a word length of 3. When using the BLAST and Gapped BLAST programs, the default parameters for each program are used. Specific methodology for these analysis methods is well known (http://www.ncbi.nlm.nih.gov).

In the present invention, the polynucleotides encoding polypeptides similar to Corl2 can also be prepared by introducing site-specific or random mutations into polynucleotides comprising the nucleotide sequence of SEQ ID NO: 2 or 4 by genetic modification methods known to those skilled in the art, such as PCR-based mutagenesis or cassette mutagenesis. Alternatively, sequences obtained by introducing mutations into the nucleotide sequence of SEQ ID NO: 2 or 4 can be synthesized using commercially available nucleic acid synthesizers.

In the present invention, polynucleotides encoding Corl2 and polynucleotides encoding polypeptides similar to Corl2 can be used to identify Purkinje cells. Since polynucleotides comprising the nucleotide sequence of SEQ ID NO: 2 or 4 bind complementarily to Corl2 mRNA in Purkinje cells, the cells can be identified by *in situ* hybridization or such, in which the polynucleotides of the present invention are contacted as probes with samples such as cerebellum, embryo, and so on. In particular, they can be used as an identification marker across all differentiation stages because Corl2 is expressed not only in adult but also at embryonic stages. The samples may be sections of adult tissues or whole-mount embryos. When polynucleotides encoding Corl2 and polypeptides similar to Corl2 are used as probes, they can be labeled with known labeling substances, for example, non-radioactive substances such as digoxigenin (DIG), biotin, and fluorescein, or radioactive substances, such as ³⁵S and ³³P.

In the present invention, polynucleotides encoding Corl2 and polypeptides similar to Corl2 can be used to produce Corl2 and polypeptides similar to Corl2 by genetic recombination. When polynucleotides encoding Corl2 and polypeptides similar to Corl2 are used to produce the polypeptides described above, the polynucleotides can be inserted into adequate vectors. The polypeptides described above may be expressed without any alteration or as fusion polypeptides with other polypeptides to improve their yield. Vectors can be selected according to the purpose and the translation system to be used. Prokaryotic cells, eukaryotic cells, or cell-free systems can be used in the translation system. For example, when *Escherichia coli* is used as a host in the prokaryotic cell system, polypeptides of interest can be produced by selecting adequate vectors, such as pET, pPRO, pCAL, pBAD, and pGEX. Alternatively, when insect cells or animal cells are used as host in the eukaryotic cell system, baculovirus expression systems such as AcNPV may be used. Recombinant polypeptides expressed as described above can be purified by known methods. For example, when expressed as fusion polypeptides with histidine or glutathione S-transferase (GST), the recombinant polypeptides can be purified using nickel resin column or glutathione column.

The present invention relates to partial polynucleotides that are complementary to the above-described polynucleotides of the present invention or complementary strands thereof. Such partial polynucleotides can be used as primers or probes in the methods and agents for identifying Purkinje cells as described below. These partial polynucleotides can also be used to prepare Corl2 and polypeptides similar to Corl2, and polynucleotides encoding them. The partial polynucleotides may be DNAs or RNAs. The length of the partial polynucleotides is not particularly limited, as long as they can be used as probes or primers, and may be the same as the full lengths of the above-described polynucleotides of the present invention. For example, the partial polynucleotides can be used as probes even when their nucleotide length is the same as the full lengths of the polynucleotides of the present invention. The length is generally 15 nucleotides or more. More specifically, the length is preferably 15 nucleotides or more and 50 nucleotides or less, more preferably 15 nucleotides or more and 30 nucleotides or less. If required, the above partial polynucleotides can be designed to contain parts of the nucleotide sequence of SEQ ID NO: 2 or 4 that exhibit low homology to other proteins (Corl1, or the like). For example, the partial polynucleotides may comprise part of the sequence from positions 357 to 485, 924 to 1478, 1689 to 3128, or 3321 to 3380 of the nucleotide sequence of SEQ ID NO: 2. These sequences correspond to sequences from positions 1 to 43, 190 to 374, 445 to 924, and 989 to 1008 in the amino acid sequence of SEQ ID NO: 1, respectively. The polynucleotides of SEQ ID NOs: 15, 16, 17, 18, 27, and 28 are examples of partial polynucleotides that can be used to isolate and amplify Corl2 as described in the Examples. The partial polynucleotides of the present invention can be prepared, for example, based on the nucleotide sequence of SEQ ID NO: 2 or 4 using a nucleic acid synthesizer.

The present invention relates to Corl2 and polypeptides similar to Corl2. As described above, Corl2 and polypeptides similar to Corl2 can be prepared using polynucleotides encoding the polypeptides of the present invention by known methods of producing proteins using gene recombination. Alternatively, these polypeptides can be prepared from naturally occurring proteins and polypeptides by Western blotting using the antibodies of the present invention described below as probes. Corl2 and polypeptides similar to Corl2 can be used to produce the anti-Corl2 antibodies described below.

Furthermore, the present invention relates to antibodies capable of binding to the polypeptides of the present invention. The antibodies may be any antibodies as long as they bind specifically to Corl2. The antibodies may be polyclonal or monoclonal, and may be antibody fragments such as Fab, Fab', F(ab')₂, and Fv. These antibodies can be prepared by means known to those skilled in the art. The polyclonal antibodies can be prepared by immunizing animals such as rabbits, mice, and goats using as an antigen Corl2 of the present invention, a polypeptide similar to Corl2, or a partial polypeptide thereof, and collecting peripheral blood. The monoclonal antibodies can be prepared by immunizing animals, preferably mammals, more preferably mice, rats, hamsters, guinea pigs, rabbits, cats, dogs, pigs, goats, horses, or cows; even more preferably mice, rats, hamsters, guinea pigs, or rabbits, with Corl2 of the present invention, a polypeptide similar to Corl2, or a partial polypeptide thereof as an antigen; fusing antibody-producing cells from spleens or lymph nodes of the animals with myeloma cells to prepare hybridomas; and collecting antibodies produced by the hybridomas. Partial Corl2 polypeptides to be used as an antigen include, for example, polypeptides from positions 643 to 772, 867 to 886, and 989 to 1008 in the amino acid sequence of SEQ ID NO: 1, polypeptides encoded by positions 2285 to 2669 and 2956 to 3013 in the nucleotide sequence of SEQ ID NO: 2, and polypeptides similar to these. The polypeptides also include portions of the above-described polypeptides having 5 amino acid residues or more, preferably 10 amino acid residues or more. Not only these polypeptides but also polypeptides comprising the above-described partial polypeptides or portions thereof can be used as antigens to prepare the antibodies of the present invention. Such polypeptides include, for example, polypeptides from positions 44 to 772, 44 to 886, 44 to 1008, 643 to 886, 643 to 1008, 867 to 1008, and 965 to 1008 in the amino acid sequence of SEQ ID NO: 1, or portions thereof having 5 amino acid residues or more, preferably 10 amino acid residues or more. For example, polypeptides comprising the segment from positions 836 to 924 in the amino acid sequence of SEQ ID NO: 1 can be used as suitable antigens to prepare the antibodies of the present invention.

Furthermore, polypeptides having any one of the amino acid sequences of positions 1 to 43, 190 to 374, 445 to 924, and 989 to 1008 in the amino acid sequence of SEQ ID NO: 1, and polypeptides comprising these polypeptides can be used as suitable antigens to prepare the antibodies of the present invention. In addition, portions of the polypeptides having 5 amino acid residues or more, preferably 10 amino acid residues or more, can also be used to prepare the antibodies of the present invention.

Since the antibodies of the present invention bind *via* antigen-antibody reaction to Corl2 specifically expressed in Purkinje cells, they can be used to identify Purkinje cells by immunohistochemical staining, Western blotting, and such. The antibodies of the present invention can also be used in methods for separating Purkinje cells as described below.

The present invention relates to methods for identifying Purkinje cells, which comprise the step of contacting a test sample with the partial polynucleotides of the present invention. In these identification methods, Purkinje cells in tissues or cell populations that are assumed to contain Purkinje cells, for example, cerebellum and embryo, are identified specifically by using polynucleotides of 15 nucleotides or more that are complementary to the nucleotide sequence of SEQ ID NO: 2 or 4, a complementary strand thereof, or the polynucleotide of SEQ ID NO: 15, 16, 17, 18, 27, or 28. As described above, the polynucleotides of 15 nucleotides or more that are complementary to the nucleotide sequence of SEQ ID NO: 2 or 4, a complementary strand thereof, or the polynucleotide of SEQ ID NO: 15, 16,17, 18, 27, or 28, bind complementarily to Corl2 mRNA in Purkinje cells. Corl2 is expressed not only in adult but also at embryonic stages. The identification methods of the present invention using the above-described partial polynucleotides are particularly superior in terms of the ability to identify Purkinje cells across differentiation stages including the adult stage. A specific example is *in situ* hybridization using the partial polynucleotides of the present invention as probes. More specifically, *in situ* hybridization using the partial polynucleotides of the present invention as probes comprises the steps of (a) contacting a test sample with the partial polynucleotides of the present invention labeled with a labeling substance, and (b) detecting signals from the labeling substance bound to the test sample. In this *in situ* hybridization, the test samples are sections of adult tissues or embryos, or whole-mount embryos. Sections from tissues or embryos can be fixed by known methods using paraformaldehyde or such. The partial polynucleotides of the present invention to be used as probes may be DNAs or RNAs, and their lengths are described above as "partial polynucleotide length". The partial polynucleotides of the present invention to be used as probes may be labeled with known labeling substances, for example, non-radioactive substances such as digoxigenin (DIG), biotin, and fluorescein, and radioactive substances such as ³⁵P and ³³P. Purkinje cells in tissues can be identified by placing a probe onto a glass slide fixed with a section, carrying out hybridization, and detecting signals from the probe. An alternative example of the methods of the present invention is *in situ* PCR using partial polynucleotides of the present invention as primers and/or probes. *In situ* PCR is a method in which PCR is carried out with fixed cells or tissue sections. There are two types of *in situ* PCR methods: one method amplifies genes of interest using labeled primers or nucleotides and detects the label in the amplified products (direct method); and the other method detects amplified genes of interest by *in situ* hybridization using labeled probes (indirect method). More specifically, the direct method comprises the steps of (a) contacting a test sample with the partial polynucleotides of the present invention labeled with a labeling substance and amplifying genes of interest in the test sample, and (b) detecting signals from the labeling substance in the amplified products. Meanwhile, the indirect method comprises the steps of (a) contacting a test sample with the partial polynucleotides of the present invention and amplifying genes of interest, and (b) contacting the amplified products obtained in step (a) with the partial polynucleotides of the present invention labeled with a labeling substance and detecting signals from the labeling substance. PCR may be carried out on glass slides or in liquid contained in tubes. An example of the procedure is briefly described as follows: tissues or such are fixed with paraformaldehyde or the like on glass slides, and then treated with protease for reagents to permeate; primers, labeled nucleotides, DNA polymerase, and others are added onto the glass slides; PCR is performed after covering the slides with cover films; the slides are washed after terminating the reaction. Genes of interest can be identified by detecting signals of the label. The methods of the present invention also include methods in which a polynucleotide is extracted by known methods from cell populations that are assumed to contain Purkinje cells, such as cerebellum, embryos, and culture cells, and tested by Southern or Northern hybridization using the above-described polynucleotides as probes.

The methods of the present invention can be used to test the purity of therapeutic material for treating cerebellum-related diseases or Purkinje cell-related diseases. The methods can also be used to confirm the presence of *in vitro* differentiation-induced Purkinje cells. The methods can also be used to elucidate the physiological functions of Purkinje cells, and so on.

The present invention also relates to methods for identifying Purkinje cells comprising the step of contacting a test sample with the antibodies of the present invention. The methods of the present invention are methods for specifically identifying Purkinje cells in tissues or cell populations that are assumed to contain Purkinje cells, for example, cerebellum and embryos, using antigen-antibody reaction between the antibodies of the present invention and Corl2 in Purkinje cells. Since the antibodies of the present invention bind to Corl2 expressed specifically in Purkinje cells *via* antigen-antibody reaction, they can be used to identify Purkinje cells by immunohistochemical staining or the like. More specifically, in the preset invention, the "methods for identifying Purkinje cells comprising the step of contacting a test sample with the antibodies of the present invention" comprise the steps of (a) contacting a test sample with the antibodies of the present invention labeled with a labeling substance, and (b) detecting signals from the labeling substance bound to the test sample. The methods for immunohistochemical staining are described below. The test samples include tissues such as cerebellum, embryos, and cell populations such as culture cells. Tissues, such as cerebellum, are fixed with formaldehyde or such and sliced into sections by known methods. The sections are contacted with a labeled anti-Corl2 antibody for antigen-antibody reaction, and the resulting signals are detected. Alternatively, the sections are contacted with an anti-Corl2 antibody as a primary antibody. Then, the sections are contacted with a labeled secondary antibody, and the resulting signals are detected. Since only Purkinje cells emit signals of the labeled antibody, Purkinje cells can be identified. An alternative example of the methods of the present invention is Western blotting using the antibodies of the present invention as probes. Western blotting can be carried out by known methods.

Herein, the "labeling substance" of the present invention refers to substances that are used to enable detection of the presence of the antibodies when they are linked physicochemically or the like to the antibodies. Specifically, such substances include enzymes, fluorescent substances, chemiluminescent substances, biotin, avidin, and radioisotopes. More specifically, the substances include enzymes such as peroxidase, alkaline phosphatase, β-D-galactosidase, glucose oxidase, glucose-6-phosphate dehydrogenase, alcohol dehydrogenase, malate dehydrogenase, penicillinase, catalase, apoglucoseoxidase, urease, luciferase, and acetylcholinesterase; fluorescent substances such as fluorescein isothiocyanate, phycobiliprotein, rare earth metal chelates, dansyl chloride, and tetramethylrhodamine isothiocyanate; radioisotopes such as ³H, ¹⁴C, ¹²⁵I,and ¹³¹I; biotin; avidin; and chemiluminescent substances such as acridinium derivatives.

The methods of the present invention can be used to test the purity of therapeutic material for treating cerebellum-related diseases and Purkinje cell-related diseases. The methods can also be used to elucidate the physiological functions of Purkinje cells, and so on.

The present invention also relates to separation of Purkinje cells. The methods of the present invention comprise using the antibodies of the present invention as a separation means, and contacting test samples with the antibodies. More specifically, the separation methods of the present invention comprise the steps of (a) contacting a test sample with an immobilized antibody of the present invention, and (b) separating cells bound to the antibody of the present invention from the antibody. In an embodiment, the methods of the present invention include affinity chromatography using columns in which an antibody of the present invention is immobilized. The test sample includes culture media containing cells that are prepared by known methods, such as trypsin treatment, from Purkinje cell-containing tissues such as cerebellum. Cells regenerated from neural stem cells or such by using regeneration techniques can also be a test sample. Purkinje cells can be prepared by loading the cell culture media onto an anti-Corl2 antibody-immobilized affinity column, and eluting (fractionating) the cells by altering the salt concentration, pH, amount of organic solvent, or such in the elution solution. In another embodiment, the methods include magnetic cell separation methods using the antibodies of the present invention. The anti-Corl2 antibody is immobilized onto magnetic beads, and cell culture media containing Purkinje cells as a test sample are contacted with the antibody-immobilized magnetic beads. Since in a cell population, only Purkinje cells bind to the antibody-immobilized magnetic beads, Purkinje cells can be separated. Alternatively, Purkinje cells can be separated using cell sorter after the antibodies of the present invention are labeled and contacted with Purkinje cell-containing cell populations.

The methods of the present invention can be used to prepare and purify therapeutic material for treating cerebellum-related diseases and Purkinje cell-related diseases. The methods can also be used to elucidate the physiological functions of Purkinje cells, and so on.

The present invention also relates to agents used in the methods for identifying Purkinje cells. The agents of the present invention comprise as an active ingredient either a partial polynucleotide of the present invention or an antibody of the present invention. The agents of the present invention are used in the above-mentioned identification of Purkinje cells. The agents may also be used to test cerebellum-related diseases and Purkinje cell-related diseases. The length of partial polynucleotides in these agents is described herein as the "partial polynucleotide length" above. The partial polynucleotides and antibodies of the present invention in the agents may be labeled. There is no limitation as to the formulation type of partial polynucleotides and antibodies in the agents of the present invention. For example, the agents may be liquid agents in which antibodies of the present invention are dispersed in an adequate solvent, or agents in which antibodies of the present invention are immobilized on magnetic beads or such. If needed, the agents of the present invention can be made into a kit with other agents, instructions, apparatuses, and the like used in the methods for identifying Purkinje cells.

The present invention also relates to agents used in the methods for separating Purkinje cells. The agents of the present invention comprise the antibodies of the present invention as an active ingredient. The antibodies of the present invention in the agents may be labeled. There is no limitation as to the formulation type of the antibodies in the agents of the present invention. For example, the agents may be liquid agents in which antibodies of the present invention are dispersed in an adequate solvent, or agents in which antibodies of the present invention are immobilized on magnetic beads, columns, or such. The agents of the present invention can be made into a kit, if needed. If necessary, the kits may comprise other agents, instructions, apparatuses, and the like used to carry out the above methods of separating Purkinje cells, as well as the polynucleotides or antibodies of the present invention.

All prior art documents cited herein are incorporated by reference herein.

### Examples

Hereinbelow, the present invention is specifically described with reference to Examples; however, it should not be construed as being limited thereto.

### [Example 1] Isolation of Corl1

Genes whose expression levels were different between the ventral and dorsal regions of an embryonic day 12.5 mouse mesencephalon were identified by the subtraction method (N-RDA) to isolate genes expressed specifically in fetal brain regions. One of the isolated fragments was a cDNA fragment encoding a protein with unknown function.

### 1-1. Adapter preparation

Adapters for use in N-RDA (ad2, ad3, ad4, ad5, and ad13) were prepared. Two oligonucleotides were prepared for each adapter and annealed to each other, and then adjusted to be 100 µM. The sequences of the adapters are shown below:
adapter ad2
   ad2S: cagctccacaacctacatcattccgt (SEQ ID NO: 5)
   ad2A: acggaatgatgt (SEQ ID NO: 6)
adapter ad3
   ad3S: gtccatcttctctctgagactctggt (SEQ ID NO: 7)
   ad3A: accagagtctca (SEQ ID NO: 8)
adapter ad4
   ad4S: ctgatgggtgtcttctgtgagtgtgt (SEQ ID NO: 9)
   ad4A: acacactcacag (SEQ ID NO: 10)
adapter ad5
   ad5S: ccagcatcgagaatcagtgtgacagt (SEQ ID NO: 11)
   ad5A: actgtcacactg (SEQ ID NO: 12)
adapter adl3
   adl3S: gtcgatgaacttcgactgtcgatcgt (SEQ ID NO: 13)
   ad13A: acgatcgacagt (SEQ ID NO: 14).

### 1-2. cDNA synthesis

Ventral and dorsal mesencephalon regions were cut out of day 12.5 mouse embryos obtained from Japan SLC. Total RNA was prepared using an RNeasy Mini Kit (Qiagen), and double-stranded cDNA was synthesized using a cDNA Synthesis Kit (TAKARA). After digestion with restriction enzyme *Rsa*I, adapter ad2 was added. The ad2S was used as a primer for amplifying the cDNA by 15 PCR cycles. The conditions for amplification were: incubation at 72°C for five minutes; 15 reaction cycles of 94°C for 30 seconds, 65°C for 30 seconds and 72°C for two minutes; and final incubation at 72°C for two minutes. In all cases, PCR in N-RDA was carried out using a reaction solution containing the following components:

| | |
|---|---|
| 10x ExTaq | 5 µl |
| 2.5 mM dNTP | 4 µl |
| ExTaq | 0.25 µl |
| 100 µM primer | 0.5 µl |
| cDNA | 2 µl |
| distilled water | 38.25 µl. |

### 1-3. Driver production

The cDNA of dorsal mesencephalon was amplified by adding ad2S, and was further amplified by five PCR cycles. The conditions for amplification were: incubation at 94°C for two minutes; five reaction cycles of 94°C for 30 seconds, 65°C for 30 seconds and 72°C for two minutes; and final incubation at 72°C for two minutes. The cDNA was purified using a Qiaquick PCR Purification Kit (Qiagen), and digested with *Rsa*I. 3 µg was used for each round of subtraction.

### 1-4. Tester production

The cDNA of dorsal mesencephalon was amplified by adding ad2S, and was further amplified by five PCR cycles. The conditions for amplification were: incubation at 94°C for two minutes; five reaction cycles of 94°C for 30 seconds, 65°C for 30 seconds and 72°C for two minutes; and final incubation at 72°C for two minutes. The cDNA was purified using a Qiaquick PCR Purification Kit (Qiagen), and digested with *Rsa*I*.* Adapter ad3 was added to 60 ng of the *Rsa*I-digested cDNA.

### 1-5. First round of subtraction

The tester and the driver produced in Sections 1-3 and 1-4 above were mixed, ethanol precipitated, and then dissolved in 1 µl of 1 x PCR buffer. After incubation at 98°C for five minutes, 1 µl of 1 x PCR buffer + 1 M NaCl was added. After another five minutes of incubation at 98°C, the tester and the driver were hybridized at 68°C for 16 hours.

With ad3S as a primer, the hybridized cDNA was amplified by ten PCR cycles (incubation at 72°C for five minutes; then ten reaction cycles of 94°C for 30 seconds, 65°C for 30 seconds and 72°C for two minutes). Next, the amplified cDNA was digested with Mung Bean Nuclease (TAKARA) and purified using a Qiaquick PCR Purification Kit. Then, the purified cDNA was amplified by 13 PCR cycles. The conditions for amplification were: incubation at 94°C for two minutes; 13 reaction cycles of 94°C for 30 seconds, 65°C for 30 seconds and 72°C for two minutes; and final incubation at 72°C for two minutes.

### 1-6. Normalization

1 µl of 2x PCR buffer was added to 8 ng of the cDNA amplified in the first round of subtraction. After incubating at 98°C for five minutes, 2 µl of 1x PCR buffer + 1 M NaCl was added. After another five minutes of incubation at 98°C, the cDNA was hybridized at 68°C for 16 hours.

The hybridized cDNA was digested with *Rsa*I and then purified using a Qiaquick PCR Purification Kit. The purified cDNA was then amplified by 11 PCR cycles using ad3S as the primer (incubation at 94°C for two minutes; then 11 reaction cycles of 94°C for 30 seconds, 65°C for 30 seconds and 72°C for two minutes; and final incubation at 72°C for two minutes). The PCR product was then digested with *RsaI* and adapter ad4 was then added.

### 1-7. Second Round of Subtraction

20 ng of the cDNA to which ad4 was added in Section 1-6 above was used as the tester and mixed with the driver of 1-3 above. The same subtraction procedure used in Section 1-5 above was performed. Finally, adapter ad5 was added to the cDNA following *Rsa*I digestion.

### 1-8. Third Round of Subtraction

2 ng of the cDNA to which ad5 was added in Section 1-7 above was used as the tester and mixed with the driver of 1-3 above. The same subtraction procedure used in section 1-5 above was performed. Finally, adapter ad13 was added to the cDNA following *Rsa*I digestion.

### 1-9. Fourth Round of Subtraction

2 ng of the cDNA to which ad 13 was added in Section 1-8 above was used as the tester and mixed with the driver of 1-3 above. The same subtraction procedure used in Section 1-5 above was performed. The amplified cDNA was cloned into pCRII (Invitrogen) and its nucleotide sequence was analyzed using the ABI3100 sequence analyzer (Applied Biosystems).

### [Example 2] Determination of the full-length Corl2 cDNA sequence

BLAST search was carried out using sequences of the cDNA fragments yielded by N-RDA. The search result suggested that the gene encoded a functionally unknown protein (Genbank accession No: XM_355050). However, the deposited sequence was an mRNA sequence deduced from a genomic sequence. Thus, the full-length cDNA sequence was cloned from an E12.5 mouse brain cDNA library by RACE.

From day 12.5 mouse embryos, brain region containing diencephalon, mesencephalon, and metencephalon was excised. Total RNA was prepared using the RNeasy Mini Kit (Qiagen), and cDNA was synthesized using the Superscript II Choice System (Invitrogen). After adding *BstX*I/*Eco*RI adapter (Invitrogen) to the double-stranded cDNA, the cDNA was cloned into BstXI-digested pCRII vector (Invitrogen) to prepare a cDNA library.

RACE was carried out by the following procedure. For the cDNA library DNA, first, first-round PCR was carried out using two primer pairs, Corl2 F1 (SEQ ID NO: 15)/TAU2 (SEQ ID NO: 19) and Corl2 R1 (SEQ ID NO: 17)/TAD3 (SEQ ID NO: 21). The conditions for PCR amplification were: incubation at 94°C for five minutes; 25 reaction cycles of 94°C for 30 seconds, 65°C for 30 seconds, and 72°C for 5 minutes; and final incubation at 72°C for two minutes. PCR was carried out using a reaction solution containing the following components:

| | |
|---|---|
| 10x buffer | 5 µl |
| 2.5 mM dNTP | 4 µl |
| ExTaq (TAKARA) | 0.25 µl |
| 100 µM primer | 0.5 µl |
| cDNA | 1 µl (10 ng) |
| DMSO | 3.75 µl |
| distilled water | 35 µl. |

Next, the first-round PCR products were diluted 100 times with distilled water, and second-round PCR was carried out using the diluted substance as a template. The two primer pairs used were Corl2 F2 (SEQ ID NO: 16)/TAU4 (SEQ ID NO: 20) and Corl2 R2 (SEQ ID NO: 18)/TAD4 (SEQ ID NO: 22). The conditions for PCR amplification were: incubation at 94°C for five minutes; 25 reaction cycles of 94°C for 30 seconds, 65°C for 30 seconds, and 72°C for five minutes; and final incubation at 72°C for two minutes. PCR was carried out using a reaction solution containing the following components:

| | |
|---|---|
| 10x buffer | 5 µl |
| 2.5 mM dNTP | 4 µl |
| ExTaq (TAKARA) | 0.25 µl |
| 100 µM primer | 0.5 µl |
| first-round PCR products (100-times dilution) | 1 µl |
| DMSO | 3.75 µl |
| distilled water | 35 µl. |

The amplified PCR product was cloned into pCRII (Invitrogen), and the nucleotide sequence was analyzed using the ABI3100 Sequence Analyzer (Applied Biosystems). The result demonstrated that the sequence encodes 1,008 amino acids. This gene was named Corl2. The amino acid sequence of mouse Corl2 is shown in SEQ ID NO: 1, and its nucleotide sequence is shown in SEQ ID NO: 2. The primer sequences are shown below:
Corl2 F1: ACGTCAATGGCTTACCAGACTCCAAG (SEQ ID NO: 15)
Corl2 F2: TGTTCTTCCGTGGAGGAGATGGCTTC (SEQ ID NO: 16)
Corl2 R1: CTTGAGAAGAGTGTTGGAGATCTGCG (SEQ ID NO: 17)
Corl2 R2: ATGGGAATGCCATAGAGGATCACCTG (SEQ ID NO: 18)
TAU2: GGCTTTACACTTTATGCTTCCGGCTC (SEQ ID NO: 19)
TAU4: CAGCTATGACCATGATTACGCCAAGC (SEQ ID NO: 20)
TAD3: AGGCGATTAAGTTGGGTAACGCCAGG (SEQ ID NO: 21)
TAD4: CCAGTCACGACGTTGTAAAACGACGG (SEQ ID NO: 22).

BLAST homology search was carried out for the amino acid sequence of Corl2 (SEQ ID NO: 1). In addition to the above-described mouse sequence (Genbank, accession No.: XM_355050), Corl2 mRNA and protein sequences deduced from the genomic sequences of chimpanzee (accession No.: XM_512119), rat (accession No: XM_225708), and *Gallus gallus* (chicken) (accession Nos.: XM_414700, BX950351, and BX929292) were deposited. In the putative mouse and rat amino acid sequences deposited, approximately 850 residues out of about 1,000 amino acids were correctly predicted; however, about 150 residues were missing and instead, the C terminal end had a completely different sequence. Specific data for the amino acid sequence homology are as follows.
The sequence from positions 1 to 643 of SEQ ID NO: 1 is identical to the sequence of XM_355050 deposited in NCBI.
The sequence from positions 644 to 772 of SEQ ID NO: 1 is deleted in the sequence of XM_355050 deposited in NCBI.
The sequence from positions 773 to 866 of SEQ ID NO: 1 is identical to the sequence of XM_355050 deposited in NCBI.
The sequence from positions 867 to 886 of SEQ ID NO: 1 is different from the sequence of XM_355050 deposited in NCBI.
The sequence from positions 887 to 965 of SEQ ID NO: 1 is identical to the sequence of XM_355050 deposited in NCBI.
The sequence of XM_355050 deposited in NCBI includes an insertion of 22 amino acids at position 966 of SEQ ID NO: 1.
The sequence from positions 966 to 988 of SEQ ID NO: 1 is identical to the sequence of XM_355050 deposited in NCBI.
The sequence from positions 989 to 1008 of SEQ ID NO: 1 is different from the sequence of XM_355050 deposited in NCBI. Meanwhile, data for the nucleotide sequence homology is as follows.
The sequence from positions 1 to 69 of SEQ ID NO: 2 has not been deposited in NCBI (novel).
The sequence from positions 70 to 2284 of SEQ ID NO: 2 is identical to the sequence of XM_355050 deposited in NCBI.
The sequence from positions 2285 to 2669 of SEQ ID NO: 2 is deleted in the sequence of XM_355050 deposited in NCBI.
The sequence from positions 2670 to 2955 of SEQ ID NO: 2 is identical to the sequence of XM_355050 deposited in NCBI.
The sequence from positions 2956 to 3013 of SEQ ID NO: 2 is different from the sequence of XM_355050 deposited in NCBI.
The sequence from positions 3014 to 3252 of SEQ ID NO: 2 is identical to the sequence of XM_355050 deposited in NCBI.
The sequence of XM_355050 deposited in NCBI comprises an insertion of 66 nucleotides at position 3253 of SEQ ID NO: 2.
The sequence from positions 3253 to 3320 of SEQ ID NO: 2 is identical to the sequence of XM_355050 deposited in NCBI.
The sequence of XM_355050 deposited in NCBI comprises an insertion of 211 nucleotides at position 3321 of SEQ ID NO: 2.
The sequence from positions 3321 to 3878 of SEQ ID NO: 2 is identical to the sequence of XM_355050 deposited in NCBI.
The sequence from positions 3879 to 4129 of SEQ ID NO: 2 has not been deposited in the sequence of XM_355050 deposited in NCBI.
In the amino acid sequences of chimpanzee and bird, a portion of the N terminus exhibited homology to SEQ ID NO: 1; however, the remaining sequences showed no homology. Some partial cDNA sequences for the human gene have been deposited (accession Nos.: AK027108, AL136667, CR614259, CR599998, and CR596209); however, putative amino acid sequences were not deposited. Thus, the gene was identified from the human genome sequence by BLAST search, and exons were linked together by predicting the 5' and 3' ends of introns, thereby yielding an ORF whose length is almost the same as that of mouse. The amino acid sequence was well conserved and there was no gap. Therefore, determination of the human Corl2 sequence was thought to be nearly completed. The amino acid sequence of human Corl2 is shown in SEQ ID NO: 3, and the nucleotide sequence of human Corl2 is shown in SEQ ID NO: 4.

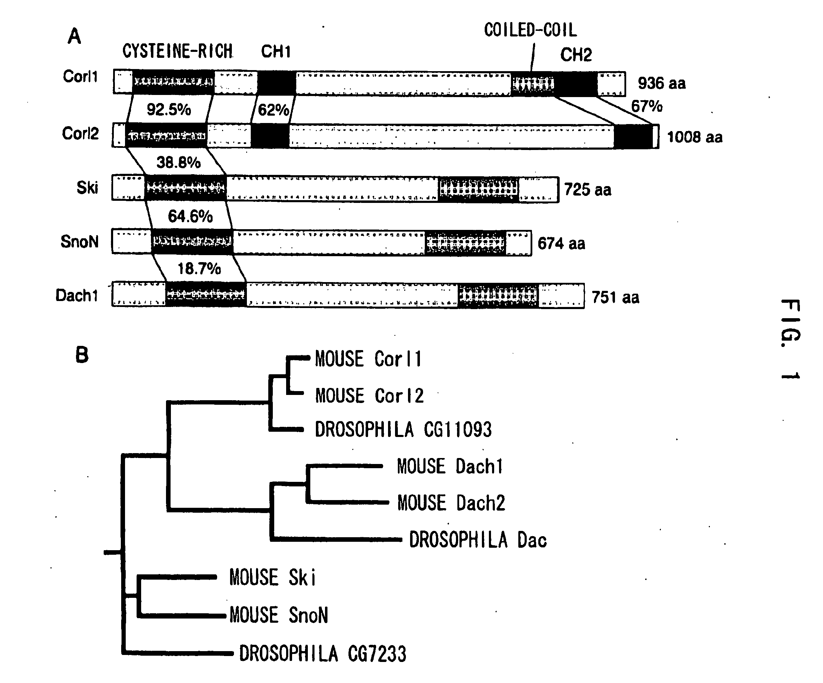

Furthermore, Corl2 exhibited an exceptionally high homology to Corl1 (GenBank accession No.: AB 185113) and was discovered to be a protein that also shows high homology to Ski, SnoN, and Dach (Fig. 1). All of them comprise a cysteine-rich domain. Furthermore, Corl1 and Corl2 comprise domains called CH1 and CH2 (Fig. 1). The locations of the cysteine-rich, CH1, and CH2 domains are listed below.

### Mouse Corl1

Cysteine-rich domain: positions 52 to 197 in the amino acid sequence of SEQ ID NO: 23

CH1 domain: positions 338 to 401 in the amino acid sequence of SEQ ID NO: 23

CH2 domain: positions 826 to 881 in the amino acid sequence of SEQ ID NO: 23

### Mouse Corl2

Cysteine-rich domain: positions 44 to 189 in the amino acid sequence of SEQ ID NO: 1

CH1 domain: positions 375 to 444 in the amino acid sequence of SEQ ID NO: 1

CH2 domain: positions 925 to 988 in the amino acid sequence of SEQ ID NO: 1

### Mouse Ski

Cysteine-rich domain: positions 101 to 260 in the amino acid sequence of SEQ ID NO: 24

### Mouse SnoN

Cysteine-rich domain: positions 143 to 302 in the amino acid sequence of SEQ ID NO: 25

### Mouse Dach1

Cysteine-rich domain: positions 184 to 352 in the amino acid sequence of SEQ ID NO: 26

The above-described Ski, SnoN, and Dach are molecules considered to be transcriptional regulatory cofactors. Ski was originally identified as an oncogene, and subsequent analyses revealed that Ski was a factor that inhibits TGFbeta signal by binding to the transcriptional regulatory factor Smad. Currently, Ski is known to bind not only to Smad but also to many other transcriptional regulatory factors, and is considered to be a factor that regulates transcriptional suppression in various phenomena such as transcriptional suppression by DNA methylation. It was originally isolated from human, and then identified from other mammals, such as mouse and rat. SnoN is assumed to have a function similar to that of Ski. Dach is thought to be a cofactor that binds to and regulates transcription with the transcriptional factors Eya and Six. It is thought that there are several Eya and Six families and they function in various tissues mainly during the developmental process. Furthermore, a Drosophila gene (Genbank accession No.: CG11093) was discovered to exhibit homology to Corl1 and Corl2 (Fig. 1), suggesting that Corl2 has an evolutionarily conserved function.

### [Example 3] Analysis of Corl2 expression

### 3-1. Analysis of Corl1 and Corl2 expression in adult mouse tissues at the gene level

The expressions of Corl1 and Corl2 in adult mouse tissues (brain, lung, liver, heart, kidney, spleen, thymus, ovary, and testis) were analyzed at the gene level by RT-PCR. The expression in an embryonic day 12.5 mouse brain was also analyzed. Single-stranded cDNA was synthesized from total RNA (Promega) of each tissue using an RNA PCR kit (TAKARA). The cDNA was used as a template. Conditions for the PCR amplification were: incubation at 94°C for two minutes; 35 reaction cycles for Corl1 and Corl2 or 25 reaction cycles for G3PDH, 94°C for 30 seconds, 65°C for 30 seconds, and 72°C for 30 seconds; and final incubation at 72°C for two minutes. PCR was carried out using the following reaction composition:

| | |
|---|---|
| 10x buffer | 1µl |
| 2.5 mM dNTP | 0.8 µl |
| ExTaq | 0.05 µl |
| 100 µM primer | 0.1 µl |
| cDNA | 1µl |
| distilled water | 7.05 µl. |

The sequences of the primers used are shown below.
Corl2 F3: GGACATGGCTTCTTCATCACAGATTC (SEQ ID NO: 27)
Corl2 R3: GTAACTGTTGCACCATCTCTTCTCGG (SEQ ID NO: 28)
Corl1 F2: ATGCAGAGAGCATCGCTAAGCTCTAC (SEQ ID NO: 29)
Corl1 R2 AAGCGGTTGGACTCTACGTCCACCTC (SEQ ID NO: 30)
G3PDH F1: ACGACCCCTTCATTGACCTCAACTAC (SEQ ID NO: 31)
G3PDH R1: CCAGTAGACTCCACGACATACTCAGC (SEQ ID NO: 32)

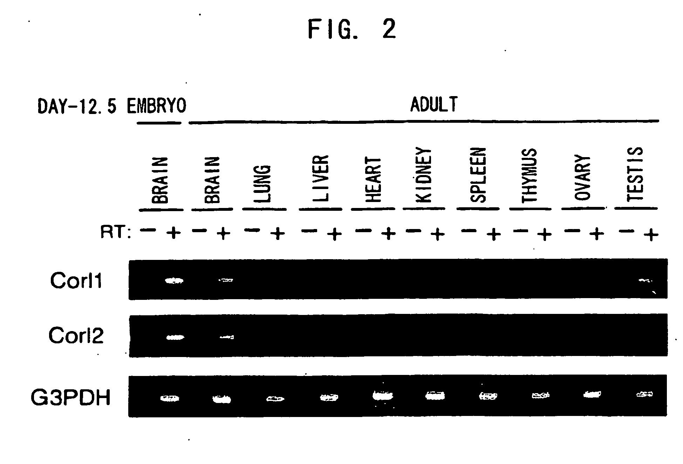

The analysis result demonstrated that in adult, Corl2 was expressed specifically in the brain (Fig. 2). It also demonstrated that the expression level in the brain was higher at fetal stages than in adult (Fig. 2).

### 3-2. Analysis of Corl2 expression in the cerebellum

The Corl2 expression in the cerebellum was analyzed at the protein level. Cerebella were obtained from day 12.5 embryos and postnatal day 12 mice.

An anti-Corl2 polyclonal antibody was prepared for carrying out the expression analysis. First, an expression vector was constructed to express a fusion protein between GST and a region of amino acids 836 to 924 amino acids of Corl2 which serves as an antigen required for immunization. After this vector was introduced into *E*. *coli* cells (JM109 strain), its expression was induced using IPTG and the fusion protein was collected using glutathione beads. Rabbits were immunized with the fusion protein several times and the blood was collected. Anti-Corl2 polyclonal antibody was obtained from the sera by affinity purification using the same GST-Corl2 antigen used in the immunization.

Immunostaining was carried out according to the protocol described below. Cerebella were isolated from day 12.5 embryos and postnatal day 12 mice, and fixed with 4% PFA/PBS(-) at 4°C for two hours. The solution was replaced with 10% sucrose/PBS(-) at 4°C for eight hours and then with 20% sucrose/PBS(-) at 4°C overnight, and then embedded in OCT. A 12 µm thick section was made. The section was placed onto a glass slide, dried at room temperature for one hour, and then it was wetted using 0.1% Triton X-100/PBS(-) for five minutes, and with PBS(-) for five minutes. It was then blocked (25% BlockAce/PBS(-)) at room temperature for 30 minutes, and reacted with a primary antibody at room temperature for one hour and further reacted at 4°C overnight. Then, the section was washed four times with 0.1% Triton X-100/PBS(-) at room temperature for ten minutes each. Then, the section was reacted with fluorescent-labeled secondary antibody at room temperature for 20 minutes. After washing in the same way, the section was washed twice with PBS(-) at room temperature for ten minutes each and the slide was then mounted. The fluorescent signal was detected with a confocal microscope. The anti-calbindin antibody and anti-RORalpha antibody were purchased from Santa Cruz.

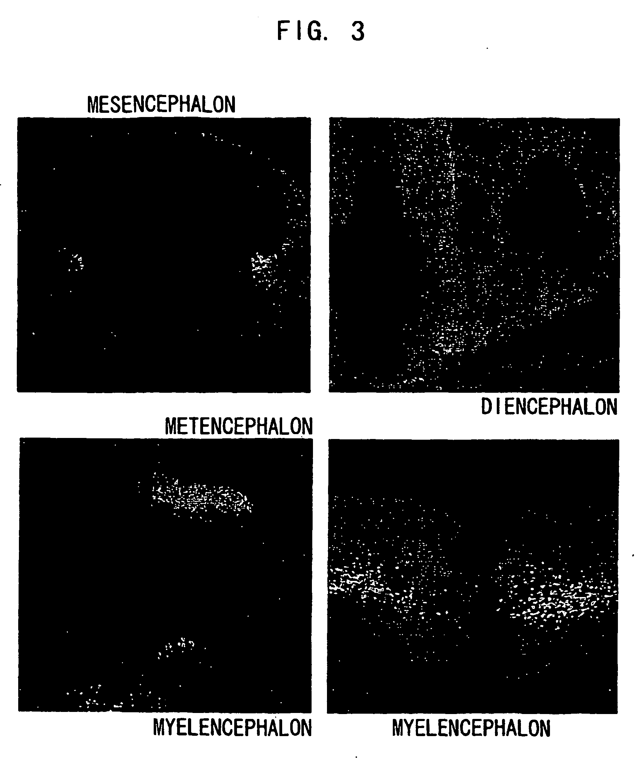

The result of immunostaining day 12.5 embryos is shown in Fig. 3. The result of immunostaining using the anti-Corl2 antibody demonstrated that Corl2 is localized in the nucleus. Furthermore, the expression pattern showed that in day 12.5 embryos, Corl2 was localized in mesencephalon, some ventral neurons of diencephalon, some dorsal neurons of metencephalon and myelencephalon, and some ventral neurons of myelencephalon. The dorsal region of metencephalon is a region in which cerebellar tissues are developed after birth, and most of the various neurons constituting the cerebellum are derived from this region. The result of BrdU administration experiment for assaying the timing of neural development demonstrated that Purkinje cells are developed in embryos around days 11 to 13. Thus, the Corl2 expression in day 12.5 embryos suggests that Corl2 is expressed in Purkinje precursor cells.

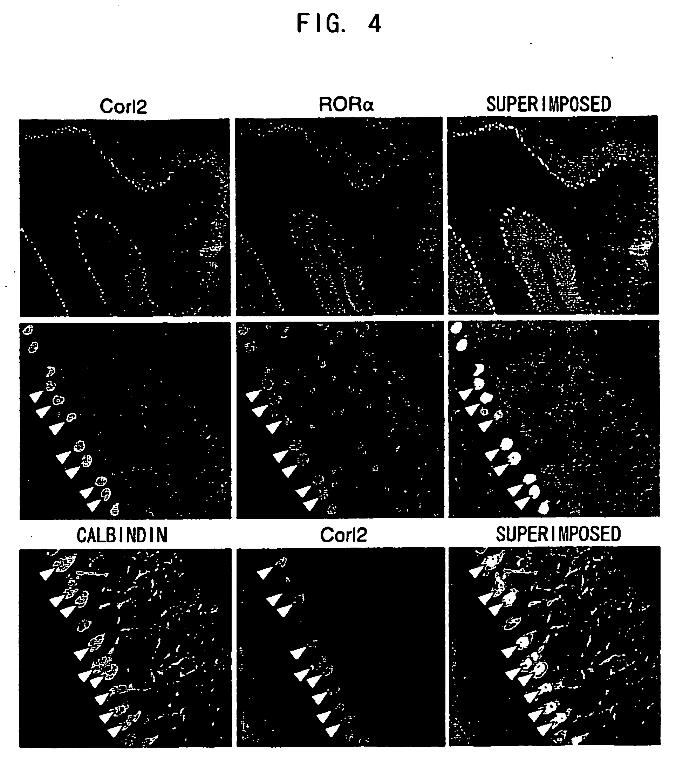

In the postnatal day 12 cerebellum, Corl2 was limitedly expressed in the Purkinje cell layer (Fig. 4; arrowheads in the photographs indicate Purkinje cells). Furthermore, all Corl2-expressing cells were co-positive for the Purkinje cell markers, calbindin and RORalpha; thus, Corl2 was demonstrated to be specifically expressed in Purkinje cells. RORalpha is known to be expressed at earlier developmental stages than calbindin. As shown in Fig. 4, RORalpha was expressed not only in Purkinje cells but also in other cells. Meanwhile, Corl2 was specific to Purkinje cells and already expressed in Purkinje cell-generating day 12.5 embryos. Thus, Corl2 was expected to be a useful marker for identifying Purkinje cells at all differentiation stages.

### Industrial Applicability

The present invention provides the novel gene Corl2. The Corl2 gene serves as a superior Purkinje cell marker, because it is highly expressed specifically in Purkinje cells. Furthermore, Corl2 can be used as a Purkinje cell marker at any differentiation stages, and is therefore highly useful for testing the purity of transplantation material, developing methods for inducing *in vitro* differentiation of Purkinje cells, and so on. Corl2 is expected to greatly contribute to promote the practical application of regenerative therapy.

## Claims

1. An isolated polynucleotide of any one of:
(a) a polynucleotide comprising the nucleotide sequence of SEQ ID NO: 2 or 4;
(b) a polynucleotide encoding a polypeptide comprising the amino acid sequence of SEQ ID NO: or 3; and
(c) a polynucleotide encoding a polypeptide comprising an amino acid sequence with an addition, deletion, insertion, and/or substitution of one or more amino acids in the amino acid sequence of SEQ ID NO:1 or 3, wherein the number of amino acids added, deleted, inserted, and/or substituted is 10% or less of the total amino acids.

2. An isolated polynucleotide of any one of:
(a) a polynucleotide encoding a polypeptide comprising the amino acid sequence of any one of positions 643 to 772, 867 to 886, and 989 to 1008 in the amino acid sequence of SEQ ID NO: 1;
(b) a polynucleotide encoding a polypeptide comprising the polypeptide encoded by the sequence of positions 2285 to 2669 or 2956 to 3013 in the nucleotide sequence of SEQ ID NO: 2;
(c) a polynucleotide encoding a polypeptide comprising an amino acid sequence with an addition, deletion, insertion, and/or substitution of one or more amino acids in a polypeptide comprising the amino acid sequence of any one of positions 643 to 772, 867 to 886, and 989 to 1008 in the amino acid sequence of SEQ ID NO: 1wherein the number of amino acids added, deleted, inserted, and/or substituted is 10% or less of the total amino acids; and
(d) a polynucleotide encoding a polypeptide comprising a polypeptide encoded by the nucleotide sequence of a polynucleotide that hybridizes under stringent conditions to a polynucleotide comprising the nucleotide sequence of positions 2285 to 2669 or 2956 to 3013 in the nucleotide sequence of SEQ ID NO: 2.

3. A vector comprising the polynucleotide of claim 1 or 2.

4. A transformant retaining the polynucleotide of claim 1 or 2 or the vector of claim 3.

5. An isolated polypeptide of any one of:
(a) a polypeptide comprising the amino acid sequence of SEQ ID NO: 1 or 3;
(b) a polypeptide comprising a polypeptide encoded by the nucleotide sequence of SEQ ID NO: 2 or 4; and
(c) a polypeptide comprising an amino acid sequence with an addition, deletion, insertion, and/or substitution of one or more amino acids in the amino acid sequence of SEQ ID NO: 1 or 3 wherein the number of amino acids added, deleted, inserted, and/or substituted is 10% or less of the total amino acids.

6. An isolated polypeptide of any one of:
(a) a polypeptide comprising the amino acid sequence of any one of positions 643 to 772, 867 to 886, and 989 to 1008 in the amino acid sequence of SEQ ID NO: 1;
(b) a polypeptide comprising a polypeptide encoded by the nucleotide sequence of positions 2285 to 2669 or 2956 to 3013 in the nucleotide sequence of SEQ ID NO: 2;
(c) a polypeptide comprising an amino acid sequence with an addition, deletion, insertion, and/or substitution of one or more amino acids in a polypeptide comprising the amino acid sequence of any one of positions 643 to 772, 867 to 886, and 989 to 1008 of SEQ ID NO: 1 wherein the number of amino acids added, deleted, inserted, and/or substituted is 10% or less of the total amino acids; and
(d) a polypeptide comprising a polypeptide encoded by the nucleotide sequence of a polynucleotide that hybridizes under stringent conditions to a polynucleotide comprising the nucleotide sequence of positions 2285 to 2669 or 2956 to 3013 in the nucleotide sequence of SEQ ID NO: 2.

7. A polynucleotide comprising at least 15 consecutive nucleotides of a nucleotide sequence from positions 357 to 458, 924 to 1478, 1689 to 3128, or 3321 to 3380 of the nucleotide sequence of SEQ ID NO: 2 or the complementary strand thereof.

8. The polynucleotide of claim 7, wherein the polynucleotide that has at least 15 consecutive nucleotides is the polynucleotide of any one of SEQ ID NOs: 15, 17, 18, 27, and 28.

9. An antibody capable of binding to the polypeptide of claim 5 or 6.

10. The antibody of claim 9, which is capable of binding to a polypeptide comprising any one of:
(a) a polypeptide comprising the amino acid sequence of any one of positions 1 to 43, 190 to 374, 445 to 924, and 989 to 1008 in the amino acid sequence of SEQ ID NO: 1; and
(b) a polypeptide comprising an amino acid sequence with an addition, deletion, insertion, and/or substitution of one or more amino acids in a polypeptide comprising the amino acid sequence of any one of positions 1 to 43, 190 to 374, 445 to 924, and 989 to 1008 in the amino acid sequence of SEQ ID NO: 1.

11. The antibody of claim 9, which is capable of binding to a polypeptide that comprises a polypeptide comprising the sequence of positions 836 to 924 of the amino acid sequence of SEQ ID NO: 1.

12. A method for producing the polypeptide of claim 5 or 6, which comprises the step of culturing the transformant of claim 4 and collecting a protein from the transformant or culture supernatant.

13. A method for identifying Purkinje cell, which comprises the step of contacting a test sample with the polynucleotide of claim 7 or 8.

14. A method for identifying Purkinje cell, which comprises the step of contacting a test sample with the antibody of any one of claims 9 to 11.

15. A method for separating Purkinje cell, which comprises the step of contacting a test sample with the antibody of any one of claims 9 to 11.

16. An agent for identifying Purkinje cell, which comprises the polynucleotide of claim 7 or 8, or the antibody of any one of claims 9 to 11.

17. An agent for separating Purkinje cell, which comprises the antibody of any one of claims 9 to 11.

18. A kit for identifying Purkinje cell, which comprises the polynucleotide of claim 7 or 8, or the antibody of any one of claims 9 to 11.

19. A kit for separating Purkinje cell, which comprises the antibody of any one of claims 9 to 11.

## Patentansprüche

1. Isoliertes Polynucleotid nach einem der Punkte:
(a) Polynucleotid, umfassend die Nucleotidsequenz der SEQ ID NO: 2 oder 4;
(b) Polynucleotid, das ein Polypeptid codiert, das die Aminosäuresequenz der SEQ ID NO: 1 oder 3 umfasst; und
(c) Polynucleotid, das ein Polypeptid codiert, umfassend eine Aminosäuresequenz mit einer Addition, Deletion, Insertion, und/oder Substitution einer oder mehrerer Aminosäuren in der Aminosäuresequenz der SEQ ID NO: 1 oder 3, wobei die Anzahl der Aminosäuren, die addiert, deletiert, inseriert, und/oder substituiert sind, 10 % oder weniger der Gesamtaminosäuren ist.

2. Isoliertes Polynucleotid nach einem der Punkte:
(a) Polynucleotid, das ein Polypeptid codiert, umfassend die Aminosäuresequenz einer der Positionen 643 bis 772, 867 bis 886 und 989 bis 1008 der Aminosäuresequenz der SEQ ID NO: 1;
(b) Polynucleotid, das ein Polypeptid codiert, umfassend das Polypeptid, welches von der Sequenz der Positionen 2285 bis 2669 oder 2956 bis 3013 in der Nucleotidsequenz der SEQ ID NO: 2 codiert wird;
(c) Polynucleotid, das ein Polypeptid codiert, umfassend eine Aminosäuresequenz mit einer Addition, Deletion, Insertion und/oder Substitution einer oder mehrerer Aminosäuren in einem Polypeptid, umfassend die Aminosäuresequenz einer der Positionen 643 bis 772, 867 bis 886 und 989 bis 1008 in der Aminosäuresequenz der SEQ ID NO: 1, wobei die Anzahl der Aminosäuren, die addiert, deletiert, inseriert, und/oder substituiert sind, 10 % oder weniger der Gesamtaminosäuren ist; und
(d) Polynucleotid, das ein Polypeptid codiert, umfassend ein Polypeptid, das von der Nucleotidsequenz eines Polynucleotids codiert wird, das unter stringenten Bedingungen an ein Polynucleotid hybridisiert, das die Nucleotidsequenz der Positionen 2285 bis 2669 oder 2956 bis 3013 in der Nucleotidsequenz der SEQ ID NO: 2 umfasst.

3. Vektor, umfassend das Polynucleotid nach Anspruch 1 oder 2.

4. Transformante, die das Polynucleotid nach Anspruch 1 oder 2 oder den Vektor nach Anspruch 3 umfasst.

5. Isoliertes Polypeptid nach einem der Punkte:
(a) Polypeptid, umfassend die Aminosäuresequenz der SEQ ID NO: 1 oder 3;
(b) Polypeptid, umfassend ein Polypeptid, das von der Nucleotidsequenz der SEQ ID NO: 2 oder 4 codiert wird; und
(c) Polypeptid, umfassend eine Aminosäuresequenz mit einer Addition, Deletion, Insertion, und/oder Substitution einer oder mehrerer Aminosäuren in der Aminosäuresequenz der SEQ ID NO: 1 oder 3, wobei die Anzahl von Aminosäuren, die addiert, deletiert, inseriert, und/oder substituiert sind, 10 % oder weniger der Gesamtaminosäuren ist.

6. Isoliertes Polypeptid nach einem der Punkte:
(a) Polypeptid, umfassend die Aminosäuresequenz einer der Positionen 643 bis 772, 867 bis 886 und 989 bis 1008 in der Aminosäuresequenz der SEQ ID NO: 1;
(b) Polypeptid, umfassend ein Polypeptid, das von der Nucleotidsequenz der Positionen 2285 bis 2669 oder 2956 bis 3013 in der Nucleotidsequenz der SEQ ID NO: 2 codiert wird;
(c) Polypeptid, umfassend eine Aminosäuresequenz mit einer Addition, Deletion, Insertion und/oder Substitution einer oder mehrerer Aminosäuren in einem Polypeptid, das die Aminosäuresequenz einer der Positionen 643 bis 772, 867 bis 886 und 989 bis 1008 der SEQ ID NO: 1 umfasst, wobei die Anzahl an Aminosäuren, die addiert, deletiert, inseriert und/oder substituiert sind, 10 % oder weniger der Gesamtaminosäuren ist; und
(d) Polypeptid, umfassend ein Polypeptid, das von der Nucleotidsequenz eines Polynucleotids codiert wird, welches unter stringenten Bedingungen an ein Polynucleotid hybridiert, das die Nucleotidsequenz der Positionen 2285 bis 2669 oder 2956 bis 3013 der Nucleotidsequenz der SEQ ID NO: 2 umfasst.

7. Polynucleotid, umfassend mindestens 15 aufeinanderfolgende Nucleotide einer Nucleotidsequenz von den Positionen 357 bis 458, 924 bis 1478, 1689 bis 3128 oder 3321 bis 3380 der Nucleotidsequenz der SEQ ID NO: 2, oder der komplementäre Strang davon.

8. Polynucleotid nach Anspruch 7, wobei das Polynucleotid, welches mindestens 15 aufeinanderfolgende Nucleotide hat, das Polynucleotid nach einer der SEQ ID NO: 15, 17, 18, 27 und 28 ist.

9. Antikörper, der zur Bindung an das Polypeptid nach Anspruch 5 oder 6 fähig ist.

10. Antikörper nach Anspruch 9, der zur Bindung an ein Polypeptid nach einem der Punkte fähig ist:
(a) Polypeptid, umfassend die Aminosäuresequenz einer der Positionen 1 bis 43, 190 bis 374, 445 bis 924 und 989 bis 1008 in der Aminosäuresequenz von SEQ ID NO: 1; und
(b) Polypeptid, umfassend eine Aminosäuresequenz mit einer Addition, Deletion, Insertion, und/oder Substitution einer oder mehrerer Aminosäuren in einem Polypeptid, umfassend die Aminosäuresequenz einer der Positionen 1 bis 43, 190 bis 374, 445 bis 924 und 989 bis 1008 in der Aminosäuresequenz der SEQ ID NO: 1.

11. Antikörper nach Anspruch 9, der zur Bindung an ein Polypeptid fähig ist, umfassend ein Polypeptid, das die Sequenz der Positionen 836 bis 924 der Aminosäuresequenz der SEQ ID NO: 1 umfasst.

12. Verfahren zur Herstellung des Polypeptids nach Anspruch 5 oder 6, umfassend den Schritt der Züchtung der Transformante nach Anspruch 4 und des Erhalts eines Proteins aus der Transformante oder dem Kulturüberstand.

13. Verfahren zur Identifizierung einer Purkinje-Zelle, umfassend den Schritt des Inkontaktbringens einer Testprobe mit dem Polynucleotid nach Anspruch 7 oder 8.

14. Verfahren zur Identifizierung einer Purkinje-Zelle, umfassend den Schritt des Inkontaktbringens einer Testprobe mit dem Antikörper nach einem der Ansprüche 9 bis 11.

15. Verfahren zur Abtrennung einer Purkinje-Zelle, umfassend den Schritt des Inkontaktbringens einer Testprobe mit dem Antikörper nach einem der Ansprüche 9 bis 11.

16. Mittel zur Identifizierung einer Purkinje-Zelle, welches das Polynucleotid nach Anspruch 7 oder 8 oder den Antikörper nach einem der Ansprüche 9 bis 11 umfasst.

17. Mittel zur Abtrennung einer Purkinje-Zelle, das den Antikörper nach einem der Ansprüche 9 bis 11 umfasst.

18. Kit zur Identifizierung einer Purkinje-Zelle, welches das Polynucleotid nach Anspruch 7 oder 8 oder den Antikörper nach einem der Ansprüche 9 bis 11 umfasst.

19. Kit zur Abtrennung einer Purkinje-Zelle, das den Antikörper nach einem der Ansprüche 9 bis 11 umfasst.

## Revendications

1. Polynucléotide isolé choisi :
(a) un polynucléotide comprenant la séquence de nucléotides de SEQ ID N°2 ou 4 ;
(b) un polynucléotide codant un polypeptide comprenant la séquence d'acides aminés de SEQ ID N°1 ou 3 ; et
(c) un polynucléotide codant un polypeptide comprenant une séquence d'acides aminés par addition, délétion, insertion et/ou substitution d'un ou de plusieurs acides aminés dans la séquence d'acides aminés de SEQ ID N°1 ou 3, où le nombre d'acides aminés ajoutés, délétés, insérés et/ou substitués est de 10 % ou moins des acides aminés totaux.

2. Polynucléotide isolé choisi :
(a) un polynucléotide codant un polypeptide comprenant la séquence d'acides aminés de l'une quelconque des positions 643 à 772, 867 à 886 et 989 à 1008 dans la séquence d'acides aminés de la SEQ ID N°1 ;
(b) un polynucléotide codant un polypeptide comprenant le polypeptide codé par la séquence des positions 2285 à 2669 ou 2956 à 3013 dans la séquence de nucléotides de la SEQ ID N°2 ;
(c) un polynucléotide codant un polypeptide comprenant une séquence d'acides aminés par addition, délétion, insertion et/ou substitution d'un ou de plusieurs acides aminés dans un polypeptide comprenant la séquence d'acides aminés de l'une quelconque des positions 643 à 772, 867 à 886 et 989 à 1008 dans la séquence d'acides aminés de SEQ ID N°1, où le nombre d'acides aminés ajoutés, délétés, insérés et/ou substitués est de 10 % ou moins des acides aminés totaux ; et
(d) un polynucléotide codant un polypeptide comprenant un polypeptide codé par la séquence de nucléotides d'un polynucléotide qui hybride dans des conditions stringentes un polynucléotide comprenant la séquence de nucléotides des positions 2285 à 2669 ou 2956 à 3013 dans la séquence de nucléotides de la SEQ ID N°2.

3. Vecteur comprenant le polynucléotide de la revendication 1 ou 2.

4. Transformant conservant le polynucléotide de la revendication 1 ou 2 ou le vecteur de la revendication 3.

5. Polypeptide isolé d'un quelconque parmi :
(a) un polypeptide comprenant la séquence d'acides aminés de SEQ ID N°1 ou 3 ;
(b) un polypeptide comprenant un polypeptide codé par la séquence de nucléotides de SEQ ID N°2 ou 4 ; et
(c) un polypeptide comprenant une séquence d'acides aminés par addition, délétion, insertion et/ou substitution d'un ou de plusieurs acides aminés dans la séquence d'acides aminés de SEQ ID N°1 ou 3, où le nombre d'acides aminés ajoutés, délétés, insérés et/ou substitués est de 10 % ou moins des acides aminés totaux.

6. Polypeptide isolé d'un quelconque parmi :
(a) un polypeptide comprenant la séquence d'acides aminés de l'une quelconque des positions 643 à 772, 867 à 886 et 989 à 1008 dans la séquence d'acides aminés de la SEQ ID N°1 ;
(b) un polypeptide comprenant un polypeptide codé par la séquence de nucléotides des positions 2285 à 2669 ou 2956 à 3013 dans la séquence de nucléotides de la SEQ ID N°2 ;
(c) un polypeptide comprenant une séquence d'acides aminés par addition, délétion, insertion et/ou substitution d'un ou de plusieurs acides aminés dans un polypeptide comprenant la séquence d'acides aminés de l'une quelconque des positions 643 à 772, 867 à 886 et 989 à 1008 dans la séquence d'acides aminés de SEQ ID N°1, où le nombre d'acides aminés ajoutés, délétés, insérés et/ou substitués est de 10 % ou moins des acides aminés totaux ; et
(d) un polypeptide comprenant un polypeptide codé par la séquence de nucléotides d'un polynucléotide qui hybride dans des conditions stringentes un polynucléotide comprenant la séquence de nucléotides des positions 2285 à 2669 ou 2956 à 3013 dans la séquence de nucléotides de la SEQ ID N°2.

7. Polynucléotide comprenant au moins 15 nucléotides consécutifs d'une séquence de nucléotides allant des positions 357 à 458, 924 à 1478, 1689 à 3128 ou 3321 1 à 3380 de la séquence de nucléotides de la SEQ ID N°2 ou du brin complémentaire de celle-ci.

8. Polynucléotide selon la revendication 7, dans lequel le polynucléotide qui a au moins 15 nucléotides consécutifs est le polynucléotide de l'une quelconque des SEQ ID N°15, 17, 18, 27 et 28.

9. Anticorps capable de liaison au polypeptide de la revendication 5 ou 6.

10. Anticorps selon la revendication 9, qui est capable de liaison au polypeptide comprenant l'un quelconque parmi :
(a) un polypeptide comprenant la séquence d'acides aminés de l'une quelconque des positions 1 à 43, 190 à 374, 445 à 924, et 989 à 1008 dans la séquence d'acides aminés de SEQ ID N°1 ; et
(b) un polypeptide comprenant une séquence d'acides aminés par addition, délétion, insertion et/ou substitution d'un ou de plusieurs acides aminés dans un polypeptide comprenant la séquence d'acides aminés de l'une quelconque des positions 1 à 43, 190 à 374, 445 à 924, et 989 à 1008 dans la séquence d'acides aminés de SEQ ID N°1.

11. Anticorps selon la revendication 9, qui est capable de liaison à un polypeptide qui comprend un polypeptide comprenant la séquence des positions 836 à 924 de la séquence d'acides aminés de SEQ ID N°1.

12. Procédé de production du polypeptide selon la revendication 5 ou 6, qui comprend l'étape consistant à cultiver le transformant de la revendication 4 et collecter une protéine à partir du transformant ou du surnageant de culture.

13. Procédé d'identification de la cellule de Purkinje, qui comprend l'étape consistant à mettre en contact un échantillon à tester avec le polynucléotide de la revendication 7 ou 8.

14. Procédé d'identification de la cellule de Purkinje, qui comprend l'étape consistant à mettre en contact un échantillon à tester avec l'anticorps de l'une quelconque des revendications 9 à 11.

15. Procédé de séparation de la cellule de Purkinje, qui comprend l'étape consistant à mettre en contact un échantillon à tester avec l'anticorps de l'une quelconque des revendications 9 à 11.

16. Agent permettant d'identifier la cellule de Purkinje, qui comprend le polynucléotide de la revendication 7 ou 8, ou l'anticorps de l'une quelconque des revendications 9 à 11.

17. Agent permettant de séparer la cellule de Purkinje, qui comprend l'anticorps de l'une quelconque des revendications 9 à 11.

18. Kit d'identification de la cellule de Purkinje, qui comprend le polynucléotide de la revendication 7 ou 8, ou l'anticorps de l'une quelconque des revendications 9 à 11.

19. Kit de séparation de la cellule de Purkinje, qui comprend l'anticorps de l'une quelconque des revendications 9 à 11.
